# EUROPEAN PATENT SPECIFICATION

(11) **EP 2 765 187 B1**
(45) Date of publication and mention of the grant of the patent: **08.08.2018**
(21) Application number: 12838198.5
(22) Date of filing: 03.10.2012
(51) Int. Cl.: C12N 5/0789, A61K 35/14, A61P 7/04, C12N 5/0735, C12N 5/078, C12N 5/10, C07D 333/38, A61K 35/15, A61K 35/19, A61K 35/28, C07D 333/40, C07D 409/14

(54) **METHOD FOR PRODUCING MEGAKARYOCYTES AND/OR PLATELETS FROM PLURIPOTENT STEM CELLS**
VERFAHREN ZUR HERSTELLUNG VON MEGAKARYOZYTEN UND/ODER BLUTPLÄTTCHEN AUS PLURIPOTENTEN STAMMZELLEN
PROCÉDÉ DE PRODUCTION DE MÉGACARYOCYTES ET/OU DE PLAQUETTES À PARTIR DE CELLULES SOUCHES PLURIPOTENTES

(30) Priority: 03.10.2011 JP 2011219545
(43) Date of publication of application: 13.08.2014
(73) Proprietor: Nissan Chemical Industries, Ltd., Chiyoda-ku Tokyo 101-0054 (JP); The University of Tokyo, Bunkyo-ku, Tokyo 113-8654 (JP)
(72) Inventor: NISHINO, Taito, Tokyo 101-0054 (JP); NAKAMURA, Takanori, Minamisaitama-gun Saitama 349-0294 (JP); IWAMOTO, Shunsuke, Funabashi-shi Chiba 274-8507 (JP); ETO, Koji, Tokyo 113-8654 (JP); NAKAUCHI, Hiromitsu, Tokyo 113-8654 (JP); TSUJI, Kayoko, Tokyo 113-8654 (JP)
(74) Representative: Blodig, Wolfgang
(86) International application number: PCT/JP2012/075705
(87) International publication number: WO 2013/051625

(56) References cited:
- EP-A1- 2 025 671
- WO-A1-2006/064957
- WO-A1-2008/041370
- WO-A1-2009/122747
- WO-A1-2010/099539
- WO-A1-2010/140685
- WO-A1-2011/034073
- WO-A1-2011/049213
- US-A1- 2009 324 583
- FUHRKEN,P.G. ET AL.: 'Tumor suppressor protein p53 regulates megakaryocytic polyploidization and apoptosis' J.BIOL.CHEM. vol. 283, no. 23, 2008, pages 15589 - 15600, XP055136725
- T. Nakamura: "A novel nonpeptidyl human c-Mpl activator stimulates human megakaryopoiesis and thrombopoiesis", Blood, vol. 107, no. 11, 1 June 2006 (2006-06-01) , pages 4300-4307, XP55047377, ISSN: 0006-4971, DOI: 10.1182/blood-2005-11-4433
- NAOYA TAKAYAMA ET AL: "Generation of functional platelets from human embryonic stem cells in vitro via ES-sacs, VEGF-promoted structures that concentrate hematopoietic progenitors", BLOOD, AMERICAN SOCIETY OF HEMATOLOGY, US, vol. 111, no. 11, 1 January 2008 (2008-01-01), pages 5298-5306, XP008158151, ISSN: 0006-4971, DOI: 10.1182/BLOOD-2007-10-117622 [retrieved on 2008-04-03]

## Description

### TECHNICAL FIELD

The present invention relates to a method for producing megakaryocytes and/or platelets from pluripotent stem cells. In particular, it relates to a method for efficiently producing megakaryocytes and/or platelets by culturing hematopoietic progenitor cells derived from iPS cells (induced pluripotent stem cells) in the presence of a compound having a platelet expanding activity.

### BACKGROUND ART

For treatment of blood-related diseases including leukemia, it is extremely important to supply therapeutically necessary amounts of blood cells stably by cell expansion. Among blood cells, platelets are essential for blood coagulation and hematostasis and, hence, are in high demand for leukemia, bone marrow transplantation, thrombocytopenia, anticancer therapy and the like. To date, platelets have been supplied from blood collected from blood donors. However, it is sometimes difficult to supply platelets to patients stably by blood donation from donors because of the risk of virus transmission, the chronic shortage of donors and the inviability of collected platelets during long term storage. Apart from blood donation from donors , other approaches such as administration of thrombopoietin (TPO) to patients and differentiation of megakaryocytes in umbilical cord blood or myelocytes were attempted However, TPO administration to patients has not come into practical use because of generation of antibodies neutralizing TPO after TPO administration. In recent years, ex vivo platelet production techniques have been studied to replace blood transfusion by returning platelets produced ex vivo by culturing hematopoietic stem cells and hematopoietic progenitor cells into living bodies. Development of these techniques into ex vivo production of large amounts of platelets is expected to dispense with the current blood donation system and almost solve the problems of the shortage of platelet products and the virus risk. Though as the source of hematopoietic stem cells and hematopoietic progenitor cells, bone marrow, umbilical cord blood and peripheral blood are known, it is difficult to stably produce and supply large amounts of platelets from these sources, because hematopoietic stem cells and hematopoietic progenitor cells which can produce megakaryocytes and platelets can be obtained only in small numbers from these sources.

In recent years, for ex vivo platelet production, several reports have been made on efficient differentiation of hematopoietic stem cells and hematopoietic progenitor cells derived from ES cells (Embryonic stem cells) into magakaryocytes and platelets. Eto et al. demonstrated that coculture with OP9 stromal cells induces mouse ES cells to differentiate into megakaryocytes (Non-Patent Document 1). Fujimoto et al. reported that they confirmed induction of platelets by using the same system as Eto et al. (Non-Patent Document 2). Successful induction of differentiation of primate ES cells into megakaryocytes (Non-Patent Document 3) and successful induction of platelets from human ES cells (Non-Patent Document 4) were reported. However, even if production of platelets from ES cells is established to a clinically applicable level, transfusion of ES cell-derived platelets to patents still has the problem of human leukocyte antigen (HLA) compatibility (in the cases of frequent transfusions into the same patient, though not in the case of the initial transfusion).

iPS cells (Induced pluripotent stem cells) are also called artificial pluripotent stem cells or induced pluripotent stem cells and are cells derived from somatic cells such as fibroblasts which have acquired pluripotency equivalent to that of ES cells by transduction of several transcription factor genes. Mouse iPS cells were established for the first time by Yamanaka et al. by transduction of four genes, Oct3/4, Sox2, Klf4 and c-Myc, into mouse fibroblasts, using the expression of Nanog gene important for maintenance of pluripotency as a marker (Non-Patent Document 5). Later, establishment of mouse iPS cells by similar methods was reported (Non-Patent Document 6 and Non-Patent Document 7). Further, it was reported that iPS cells were established by transduction of only the three genes other than c-Myc (Oct3/4, Sox2 and Klf4) to solve the problem of tumorigenesis of iPS cells (Non-Patent Document 8). With respect to human iPS cells, Thomson et al. established human iPS cells by transduction of OCT3/4, SOX2, NANOG and LIN28 into human fibroblasts (Non-Patent Document 9). Yamanaka et al. also established human iPS cells by transduction of OCT3/4, SOX2, KLF4 and c-MYC into human fibroblasts (Non-Patent Document 9). iPS cells are expected to solve the problems with ex vivo platelet production such as insufficient quantities of hematopoietic stem cells and hematopoietic progenitor cells in bone marrow and umbilical cord blood, ethical issues and the problem of rejection in terms of using ES cells. In a study made from such a perspective, success in induction of differentiation of human iPS cells into platelets was reported (Patent Document 1), and addition of proteins such as TPO is effective for induction of differentiation into megakaryocytes and platelets is suggested.

Recent years have seen reports that low-molecular-weight compounds synthesized through organic chemistry are effective as therapeutic drugs for thrombocytopenia (Patent Documents 2 and 3) and effective for ex vivo expansion of hematopoietic stem cells (Patent Documents 4, 5, 6, 7 and 8).

EP 2025671 discloses compounds effective for preventing, treatment or improving diseases against which activation of the thrombopoietin receptor is effective. WO2010/099539 A1 discloses methods for the in vitro maintenance, expansion, culture, and/or differentiation of pluripotent cells.
US2009/324583 discloses a method for inducing the differentiation of CD34+ hematopoietic stem cells into megakaryocytes and platelets.
WO2008/041370 A1 discloses a net-like structure enclosing hematopoietic progenitor cells, a method for preparing the net-like structure, and a method for efficiently preparing blood cells such as mature megakaryocytes and platelets from the net-like structure.
WO2006064957 A1 discloses compounds effective in the prevention, treatment, or alleviation of diseases in which thrombopoietin receptor activation is effective. T. Nakamura et al., Blood 2006, 107: 4300-4307 discloses a novel nonpeptidyl human c-Mpl activator that stimulates human megakaryopoiesis and thrombopoiesis.

### PRIOR ART DOCUMENT

### PATENT DOCUMENTS

Patent Document 1: WO 2009/122747
Patent Document 2: WO 2004/108683
Patent Document 3: WO 2007/010954
Patent Document 4: WO 2009/072624
Patent Document 5: WO 2009/072625
Patent Document 6: WO 2009/072626
Patent Document 7: WO 2009/072635
Patent Document 8: WO 2010/140685

### NON-PATENT DOCUMENTS

Non-Patent Document 1: Eto et al., Proc. Acad. Sci. USA 2002, 99: 12819-12824.
Non-Patent Document 2: Fujimoto et al., Blood 2003, 102: 4044-4051.
Non-Patent Document 3: Hiroyama et al. Exp. Hematol. 2006, 34: 760-769.
Non-Patent Document 4: Takayama et al., Blood 2008, 111: 5298-5306.
Non-Patent Document 5: Okita et al., Nature 2007, 448: 313-317.
Non-Patent Document 6: Wernig et al., Nature 2007, 448: 318-324.
Non-Patent Document 7: Maherali et al., Cell Stem Cell 2007, 1: 55-70.
Non-Patent Document 8: Nakagawa et al., Nat Biotechnol 2008, 26: 101-106.
Non-Patent Document 9: Yu et al., Science 2007, 318: 1917-1920.
Non-Patent Document 10: Takahashi et al., Cell 2007, 131: 861-872.

### DISCLOSURE OF THE INVENTION

### TECHNICAL PROBLEM

An object of the present invention is to establish a method for obtaining megakaryocytes and platelets from pluripotent stem cells, in particular, to establish a method for obtaining megakaryocytes and platelets with stable efficiency.

### SOLUTION TO PROBLEMS

The present inventors have conducted intensive studies to solve the above-mentioned object in search for compounds capable of inducing megakaryopoiesis and thrombopoiesis from pluripotent stem cells and found out that the compounds represented by the following formula (I) have excellent megakaryopoietic and thrombopoietic activity even in the absence of TPO and that megakaryocytes and/or platelets can be produced ex vivo stably and efficiently. The present invention was accomplished on the basis of this discovery.

Namely, the present invention provides the methods of claims 1 to 13.

### ADVANTAGEOUS EFFECT(S) OF INVENTION

The present invention makes it possible to induce megakaryocytes and platelets from hematopoietic progenitor cells derived from pluripotent stem cells (human iPS cells) by using the compounds represented by the formula (I), tautomers, prodrugs or pharmaceutically acceptable salts of the compounds or solvates thereof (which will be collectively referred to as specific compounds). When used in culture of hematopoietic progenitor cells derived from pluripotent stem cells, the specific compounds induce megakaryocytes and platelets more stably and more efficiently than proteins such as TPO. Namely, the method of the present invention realizes stable blood preparations containing platelets as an active ingredient.

The specific compounds are low-molecular-weight compounds obtainable by ordinary processes for organic synthesis and hence, are easy to produce under conditions which preclude microbial cell survival. Therefore, the method for producing platelet using the specific compounds can prevent contamination with an unknown pathogen or a biomaterial from an nonhuman animal more easily than conventional methods using proteins such as cytokines and growth factors obtained by gene recombination technology. Namely, platelets produced by the method of the present invention can avoid infections, contamination with foreign genes or immune response to foreign proteins. While being proteins, cytokines and growth factors can be stored or used within very narrow optimal ranges in terms of pH, temperature and ion strength, the specific compounds can be used and stored under relatively broad ranges of conditions. In addition, because the specific compounds can be produced continuously at low costs, unlike proteins, it is possible to eventually reduce treatment cost.

### DESCRIPTION OF DRAWING(S)

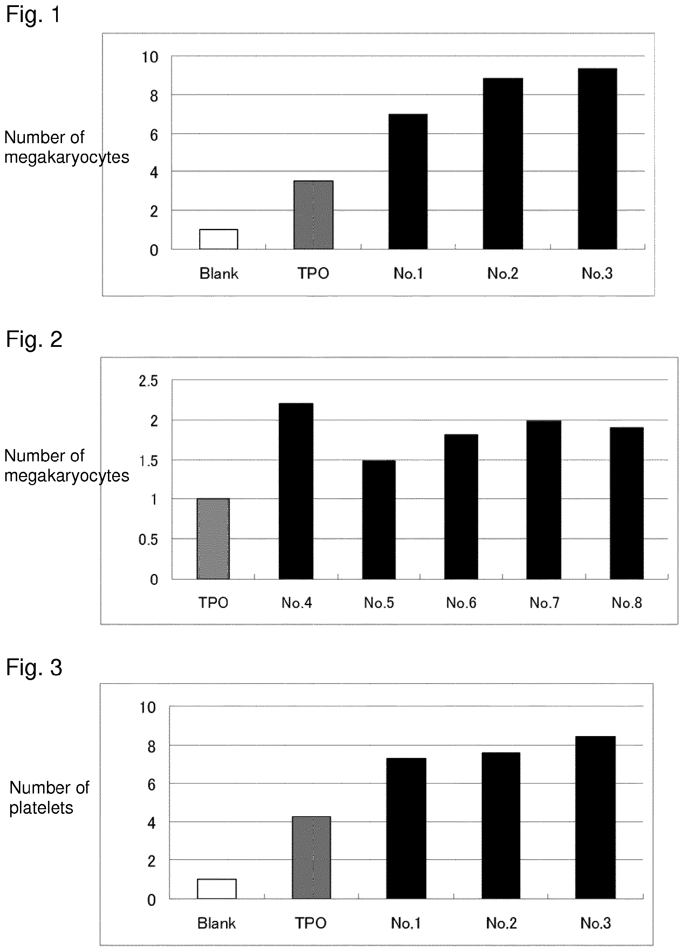

[Fig. 1] A graph showing that megakaryocytes (CD41a⁺ CD42b⁺ cells) were expanded more remarkably in a culture of iPS cell-induced hematopoietic progenitor cells in the presence of specific compounds than in the presence of TPO. The ordinance of the graph is the number of megakaryocytes (CD41a⁺ CD42b⁺ cells) in the presence of the specific compounds relative to that in the absence of the compounds.
[Fig. 2] A graph showing megakaryocytes (CD41a⁺ CD42b⁺ cells) were expanded more remarkably in a culture of iPS cell-induced hematopoietic progenitor cells in the presence of specific compounds than in the presence of TPO. The ordinance of the graph is the number of megakaryocytes (CD41a⁺ CD42b⁺ cells) in the presence of the specific compounds relative to that in the presence of TPO.
[Fig. 3] A graph showing that platelets (CD41a⁺ CD42b⁺ cells) were expanded more remarkably in a culture of iPS cell-derived hematopoietic progenitor cells in the presence of specific compounds than in the presence of TPO. The ordinance of the graph is the number of platelets (CD41a⁺ CD42b⁺ cells) in the presence of the specific compounds relative to that in the absence of the compounds.

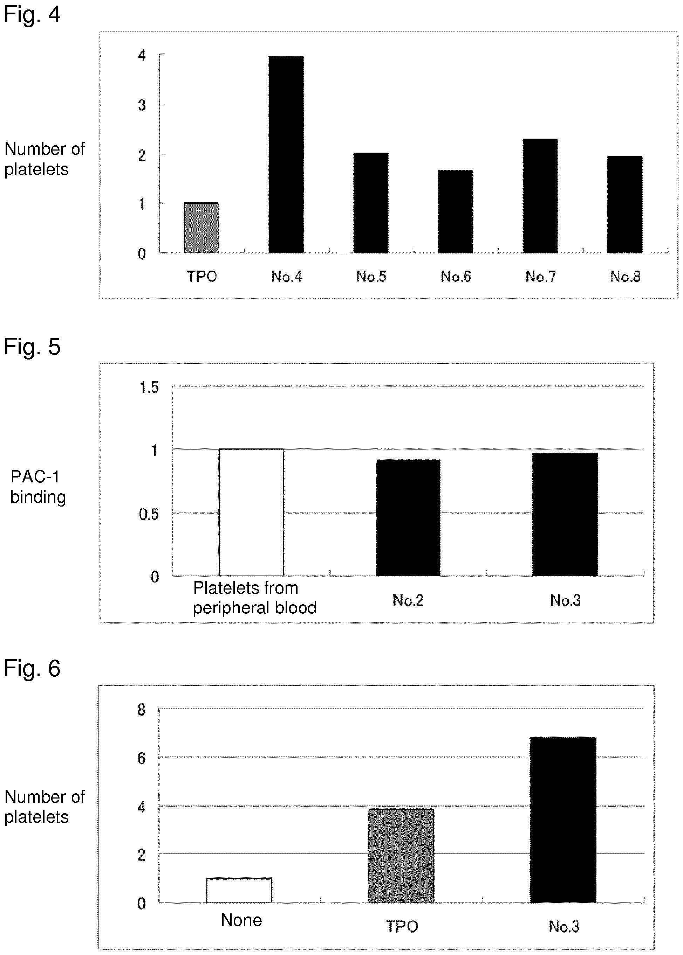

[Fig. 4] A graph showing platelets (CD41a⁺ CD42b⁺ cells) were expanded more remarkably in a culture of iPS cell-derived hematopoietic progenitor cells in the presence of specific compounds than in the presence of TPO. The ordinance of the graph is the number of platelets (CD41a⁺ CD42b⁺ cells) in the presence of the specific compounds relative to that in the presence of TPO.
[Fig. 5] A graph showing integrin activation (PAC-1 binding to platelets) by ADP on platelets (CD41a⁺ CD42b⁺ cells) prepared from iPS cells in the presence of specific compounds. The ordinate of the graph is the PAC-binding to the platelets relative to the PAC-binding to platelets from peripheral blood.
[Fig. 6] A graph showing platelets (CD41a⁺ CD42b⁺ cells) were expanded more remarkably in a culture of ES cell-derived hematopoietic progenitor cells in the presence of a specific compound than in the presence of TPO. The ordinance of the graph is the number of platelets (CD41a⁺ CD42b⁺ cells) in the presence of the specific compounds relative to that in the absence of the specific compound.

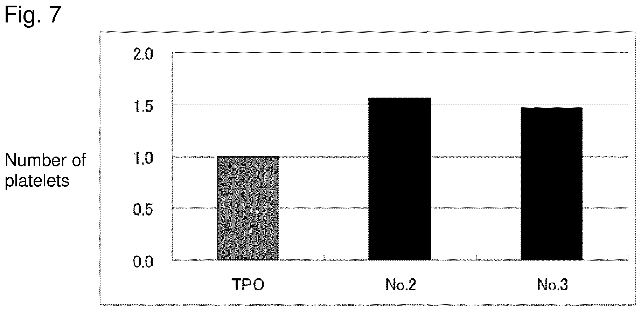

[Fig. 7] A graph showing platelets (CD41a⁺ CD42b⁺ cells) were expanded more remarkably in a culture of genetically manipulated hematopoietic progenitor cells with enhanced proliferative and differentiative capability in the presence of a specific compound than in the presence of TPO. The ordinance of the graph is the number of platelets (CD41a⁺ CD42b⁺ cells) in the presence of the specific compound relative to that in the presence of TPO.

### DESCRIPTION OF EMBODIMENT(S)

Now, the present invention will be described in detail.

The terms herein are defined as follows.

Pluripotent stem cells are cells having both pluripotency which allows them to differentiate into various kinds of cells in the body such as those in the endoderm (interior stomach lining, gastrointestinal tract, the lungs), in the mesoderm (muscle, bone, blood, urogenital) and in the ectoderm (epidermal tissues and nervous system) and self-renewal ability to proliferate through cell division while maintaining the pluripotency, and as examples , ES cells, iPS cells, embryonic germ cells (EG cells) and Muse cells may be mentioned. ES cells are pluripotent stem cells derived from an embryo at an early stage in the development of animals called the blastocysto stage. iPS cells are also called artificial pluripotent stem cells or induced pluripotent stem cells and are cells derived from somatic cells such as fibroblasts which have acquired pluripotency equivalent to that of ES cells by transduction of several transcription factor genes. EG cells are pluripotent stem cells derived from spermatogonial cells (see: Nature. 2008, 456, 344-49). Muese cells are pluripotent stem cells separated from mesenchymal cell populations (see: Proc Natl Acad Sci USA. 2010, 107, 8639-43).

Hematopoietic stem cells are defined as cells having both pluripotency which allows them to differentiate into blood cells of all lineages and the ability to renew themselves while maintaining the pluripotency. Multipotential hematopoietic progenitor cells are cells which can differentiate into a plurality of blood cell lineages, though not into all blood cell lineages Unipotential hematopoietic progenitor cells are cells which can differentiate into only one blood cell lineage.

Hematopoietic progenitor cells are a group of cells which covers both pluripotent hematopoietic progenitor cells and unipotent hematopoietic progenitor cells. For example, the hematopoietic progenitor cells in the present invention may be granulocyte-macrophage colony forming cells (CFU-GM), eosinophil colony forming cells (EO-CFC), erythroid burst forming cells (BFU-E) as erythroid progenitor cells, megakaryocyte colony forming cells (CFU-MEG), megakaryocyte progenitor cells, megakaryoblasts, promegakaryocytes, megakaryocyte/erythroid progenitor cells (MEP cells) or myeloid stem cells (mixed colony forming cells, CFU-GEMM). Among them, hematopoietic progenitor cells which differentiate into megakaryocytes and platelets are megakaryocyte colony forming cells (CFU-MEG), megakaryocyte progenitor cells, megakaryoblasts, promegakaryocytes, megakaryocyte/erythroid progenitor cells (MEP cells) and myeloid progentor cells (mixed colony forming cells, CFU-GEMM).

Megakaryocytes are differentiated cells which develop through myeloid progenitor cells, MEP cells, megakaryocyte progenitor cells, megakaryoblasts and promegakaryocytes with an increase in cell size during the cytoplasmic maturation events such as polyploidization, development of the demarcation membrane system and granulation and have the potential to produce platelets through formation of proplatelet processes.

Platelets are anucleate cells derived from megakaryocytes and play an important role in blood coagulation.

CD41a⁺ cells are means expressing CD (cluster of differentiation) 41a antigen on the cell surface. Likewise, CD42b⁺ cells are means expressing CD 42b antigen on the cell surface. These antigens are markers for megakaryocytes and platelets. Populations of CD41a⁺ and CD42b⁺ cells are enriched with megakaryocytes and platelets.

In the present invention, differentiation of hematopoietic progenitor cells means conversion of hematopoietic progenitor cells to mature blood cells having specific functions such as erythrocytes, leukocytes, megakaryocytes and platelets.

The specific compounds to be used in the present invention act on hematopoietic progenitor cells derived from pluripotent stem cells and have such an activity that they induce megakaryopoiesis and thrombopoiesis from such hematopoietic progenitor cells cultured ex vivo in the presence of a specific compound. Even when hematopoietic progenitor cells cannot produce megakaryocytes and platelets efficiently, use of a specific compound makes it possible to produce megakaryocytes and platelets efficiently by culturing hematopoietic progenitor cells derived from pluripotent stem cells ex vivo. Specifically speaking, it is possible to produce megakaryocytes and platelets by culturing hematopoietic progenitor cells in a medium containing a specific compound. It is also possible to produce megakaryocytes and platelets more efficiently by further adding various cytokines or growth factors, by coculturing them with feeder cells or by further adding other low-molecular-weight compounds which act on hematopoietic progenitor cells.

In the present invention, induced pluripotent stem (iPS) cells may be used as long as they have both pluripotency an self-renewal ability and can differentiate into platelets. Examples of transcription factor genes known to be necessary for imparting pluripotency in establishment of iPS cells include Nanog, Oct3/4, Sox2, Klf4, c-Myc and Lin28. iPS can be established by introducing the combination of Oct3/4, Sox2, Klf4 and c-Myc, the combination of Oct3/4, Sox2, Nanog, and Lin28 or the combination of Oct3/4, Sox2 and Klf4 selected from these genes into somatic cells such as fibroblasts. The iPS cells to be used in the present invention may be established by any methods, and in addidtion to those established by introduction of the above-mentioned genes, those established by introduction of genes other than those mentioned above or those established by using a protein or a low-molecular-weight compound may be used.

For culturing and subculturing pluripotent stem cells, a medium usually used to maintain pluripotency may be used. For example, Iscove's Modified Dulbecco's medium (IMDM), Dulbecco's Modified Eagles's Medium (DMEM), F-12 medium, X-VIVO 10 (Lonza), X-VIVO 15 (Lonza), mTeSR (Stemcell Technologies), TeSR2 (Stemcell Technologies), StemProhESC SFM (Invitrogen) and the like may be mentioned. The culture medium may be supplemented with proteins such as basic fibroblast growth factor (bFGF), insulin and transforming growth factor β (TGF-β), serum, KnockOut SR (Invitrogen), amino acids such as glutamine or 2-mercaptoethanol, and the culture vessel may be coated with an extracellular matrix such as laminins-1 to -12, collagen, fibronectin, vitronectin, Matrigel (Becton, Dickinson and Compnay) or Geltrex (Invitrogen). Pluripotent stem cells may be co-cultured with feeder cells. Any feeder cells that contribute to proliferation and maintenance of pluripotent cells may be used, and for example, C3H10T1/2 cell line, OP9 cells, NIH3T3 cells, ST2 cells, PA6 cells, preferably mouse embryonic fibroblast cells (MEF cells) or SL10 cells may be used. It is preferred to suppress growth of feeder cells, for example, by treatment with mitomycin C or irradiation before use.

Pluripotent stem cells are cultured usually at a temperature of from 25 to 39°C, preferably 33 to 39°C, in the atmosphere having a CO₂ concentration of from 4 to 10 vol%, preferably from 4 to 6 vol%.

The source of hematopoietic progenitor cells to be used in the present invention is a sac-like structure. A "sac-like structure" is an iPS or ES cell-derived three-dimensional saccular structure (having a cavity inside) formed of a population of endothelial cells or the like and containing hematopoietic progenitor cells inside. For the details of sac-like structures, see TAKAYAMA et al., BLOOD 2008, 111: 5298-5306.

For preparation of a sac-like structure (hereinafter referred to also as an iPS-sac), suitable culture conditions may be selected, and the suitable culture conditions vary depending on the organism as the source of the iPS cells to be used. For example, for human iPS cells, IMDM containing fetal bovine serum (FBS) in a final concentration of 15%, optionally supplemented with insulin, transferrin, lactoferrin, cholesterol, ethanolamine, sodium selenite, α-monothioglycerol, 2-mercaptoethanol, bovine serum albumin, sodium pyruvate, ascorbic acid, polyethylene glycol, various vitamins, various amino acids and various antibiotics, may be used as the culture medium. As factors which induce human iPS cells to form a sac-like structure, vascular endothelial growth factor (VEGF) and placental growth factor (PGF) may, for example, be mentioned, and VEGF is preferred. VEGF may be added at a concentration of about 10 ng/mL to100 ng/mL, preferably at a concentration of about 20 ng/mL. A human iPS cell culture may be incubated, for example, in 5% CO₂ at 36 to 38°C, preferably at 37°C, though the incubation conditions differ depending on the human iPS cells to be used. Further, it is possible to produce a sac-like structure more efficiently from human iPS cells by coculture with feeder cells. Any feeder cells that contribute to induction of differentiation of pluripotent stem cells into hematopoietic progenitor cells may be used, and for example, mouse embryonic fibroblast cells (MEF cells) or SL10 cells, preferably, C3H10T1/2 cell line, OP9 cell line, ST2 cells, NIH3T3 cells, PA6 cells or M15 cells , more preferably C3H10T1/2 cell line or OP9 cells may be used. It is preferred to suppress growth of feeder cells, for example, by treatment with mitomycin C or irradiation before use. The incubation time until formation of a sac-like structure differs depending on the human iPS cells used, and, for example, the presence of a sac-like structure can be confirmed 14 to 17 days after inoculation on feeder cells.

The sac-like structure thus formed has a cystic structure demarcated by septa of cells positive for a mesodermal cell marker Flk1 (fetal liver kinase 1), CD1, CD34 or UEA-1 lectin (Ulex europaeus.agglutinin-1). The inside of the sac-like structure is rich in hematopoietic progenitor cells. Before inducing hematopoietic progenitor cells to differentiate into various blood cells, it is necessary to separate hematopoietic progenitor cells from the cells from the septal cells. The separation may be attained by physical means. For example, the septal cells can be separated from the hematopoietic progenitor cells by breaking a sac-like structure with a pipette or a syringe and then passing the cells through a sterilized sieve-like tool (such as a cell strainer).

In the present invention, the hematopoietic progenitor cells isolated from a sac-like structure or the like as mentioned above are differentiated into megakaryocytes and/or platelets. Differentiation of hematopoietic progenitor cells into platelets means differentiation of hematopoietic progenitor cells into megakaryocytes and production of platelets from the megakaryocytes. Specifically speaking, hematopoietic progenitor cells derived from pluripotent stem cells are cultured under conditions suitable for induction of differentiation of megakaryocytes and/or platelets. To differentiate hematopoietic progenitor cells into megakaryocytes and/or platelets, ordinary culture media used for hematopoietic cell culture, such as Iscove's Modified Dulbecco's medium (IMDM), Dulbecco's Modified Eagles's Medium (DMEM), F-12 medium, X-VIVO 10 (Lonza), X-VIVO 15 (Lonza), McCoy's 5A medium, Eagle's MEM, αMEM, RPMI1640, StemPro34 (Invitrogen), StemSpan H3000 (Stemcell Technologies), StemSpanSFEM(Stemcell Technologies), Stemlinell(Sigma-Aldrich) or QBSF-60(Quality Biological), may be used. As supplements to the media, bovine fetal serum, human serum, horse serum, insulin, transferring, lactoferrin, cholesterol, ethanolamine, sodium selenite, α-monothioglycerol, 2-mercaptoethanol, bovine serum albumin, sodium pyruvate, ascorbic acid, polyethylene glycol, various vitamins, various amino acids, various antibiotics, agar, agarose, collagen, methylcellulose and the like may be mentioned.

"Culturing in the presence of a specific compound" means culturing in a medium containing a specific compound of the present invention, for example, in a medium containing a specific compound only or a medium containing a specific compound together with other differentiation inducing factors. As the other differentiation inducing factors, interleukin-1α (IL-1α), IL-3, IL-4, IL-5, IL-6, IL-9, IL-11, erythropoietin (EPO), granulocyte-macrophage colony-stimulating factor (GM-CSF), stem cell factor (SCF), granulocyte colony-stimulating factor (G-CSF), flk2/flt3 ligand (FL) or Heparin, or a combination of two or more of them may be mentioned. For example, differentiation into megakaryocytes and platelets can be induced in a culture containing a specific compound of the present invention (from 1 ng/mL to 1000 ng/mL, preferably from 10 ng/mL to 200 ng/mL, more preferably from 20 ng/mL to 100ng/mL), optionally supplemented with SCF (from 10 to 200 ng/mL, preferably about 50 ng/mL) and Heparin (from 10 to 100 U/mL, preferably about 25 U/mL), within about 7 to 15 days. A specific compound of the present invention may be added directly to the culture medium, or after dissolved in an appropriate solvent before use. Examples of the appropriate solvent include dimethyl sulfoxide (DMSO) and various alcohols, but it is not restricted thereto. A specific compound may be immobilized on the surface of a culture plate or a carrier.

A specific compound may be provided or stored in any forms, for example, in a solid form as a tablet, a pill, a capsule or a granule, in a liquid form as a solution or suspension in an appropriate solvent or resolvent, in the form bound to a plate or carrier. When it is formulated into such a form, additives such as a preservative like p-hydroxybenzoates; an excipient like lactose, glucose, sucrose and mannitol; a lubricant like magnesium stearate and talc; a binder like polyvinyl alcohol, hydroxypropylcellulose and gelatin, a surfactant like fatty acid esters, a plasticizer like glycerin may be added. The additives are not restricted to those mentioned above and a person skilled in the art can use any additives of choice.

The culture medium may be supplemented with one or more chemical substances effective in differentiation of hematopoietic progenitor cells into platelets (see: Schweinfurth et al., Platelets, 21: 648-657 2010, Lordier et al., Blood, 112: 3164-3174 2008). Examples of them include retinoic acid receptor ligands such as all-trans-retinoic acid, histone deacetylase inhibitors such as valproic acid, trichostatin A, SAHA (suberoylanilide hydroxamic acid) and APHA (aroyl-pyrrolyl-hydroxyamide), ROCK (Rho-associated coiled-coil forming kinase/Rho-binding kinase) inhibitors such as (R)-(+)-trans-N-(4-pyridyl)-4-(1-aminoethyl)-cyclohexanecarboxamide·2HCl·H2O (Y27632), myosin heavy chain II ATPase such as blebbistatin, myosin light chain kinase inhibitors such as ML7 and prostaglandin E2 but are not restricted thereto. Before treating cells with these compounds, a person skilled in the art may determine the optimum concentration of these chemical substances by experiment and may choose appropriate treating time and method. For example, in the case of blebbistatin as a myosin heavy chain II ATPase inhibitor, from 0.3 to 15 µg/mL, or from 1 to 10 µg/mL of blabbistatin may be added, and the incubation time is, preferably, for example, about from 3 to 10 days, particularly, about from 4 to 7 days. Alternatively, Y-27632 as a ROCK inhibitor may be used at 5 to 15 µM, or 8 to 12 µM, preferably about 10 µM, and valproic acid as a HDAC inhibitor may be used at 0.1 to 1 mM, or 0.2 to 0.7 mM, preferably about 0.5 mM. The treatment time for Y-27632 and valproic acid is about from 3 to 21 days, preferably about from 7 to 14 days.

Hematopoietic stem cells and/or hematopoietic progenitor cells are cultured usually at a temperature of from 25 to 39°C, preferably from 33 to 39°C, in the atmosphere having a CO₂ concentration of from 4 to 10 vol%, preferably from 4 to 6 vol%. Hematopoietic progenitor cells may be co-cultured with feeder cells for induction of megakaryocytes and platelets. Any feeder cells that contribute to contribute to induction of differentiation of hematopoietic progenitor cells into megakaryocytes or platelets may be used, and for example, mouse embryonic fibroblast cells (MEF cells) or SL10 cells, preferably, C3H10T1/2 cell line, OP9 cell line, ST2 cells, NIH3T3 cells, PA6 cells or M15 cells , more preferably C3H10T1/2 cell line or OP9 cells may be used. It is preferred to suppress growth of feeder cells, for example, by treatment with mitomycin C or irradiation before use.

Hematopoietic progenitor cells can be cultured in a culture vessel generally used for animal cell culture such as a Petri dish, a flask, a plastic bag, a Teflon (registered trademark) bag, optionally after preliminary coating with an extracellular matrix or a cell adhesion molecule. The material for such a coating may be collagens I to XIX, fibronectin, vitronectin, laminins 1 to 12, nitogen, tenascin, thrombospondin, von Willebrand factor, osteoponin, fibrinogen, various elastins, various proteoglycans, various cadherins, desmocolin, desmoglein, various integrins, E-selectin, P-selectin, L-selectin, immunoglobulin superfamily, Matrigel, poly-D-lysine, poly-L-lysine, chitin, chitosan, Sepharose, alginic acid gel, hydrogel or a fragment thereof. Such a coating material may be a recombinant material having an artificially modified amino acid sequence. Hematopoietic progenitor cells may be cultured by using a bioreactor which can mechanically control the medium composition, pH and the like and obtain high density culture (Schwartz RM, Proc. Natl. Acad. Sci. U.S.A., 88:6760, 1991; Koller MR, Bone Marrow Transplant, 21:653, 1998; Koller, MR, Blood, 82: 378, 1993; Astori G, Bone Marrow Transplant, 35: 1101, 2005).

When megakaryocytes and platelets are produced from human iPS cells, since the efficiency of sac-like structure production varies clone to clone, preliminary choice of iPS cell clones which produce a sac-like structure efficiently makes it possible to produce a large number of megakaryocytes and platelets more efficiently from the sac-like structures produced by the selected iPS cell clones. As the iPS cell clones producing a sac-like structure efficiently, clones forming at least 10, preferably at least 15 sac-like structures per 1×10⁵ cells may be chosen.

In addition, introduction of an oncogene or the like increases the proliferative capability of hematopoietic progenitor cells obtained from pluripotent stem cells (see: WO/2011/034073). The oncogene may, for example, be a MYC family gene (such as c-Myc, n-myc or l-myc gene), a SRC family gene, a RAS family gene, a g RAF family gene, c-kit gene, Abl gene or the like, preferably a gene of the Myc family more preferably c-Myc gene. The senescence of cells resulting from oncogene introduction can be prevented by simultaneous introduction of a polycomb gene or the like. The polycomb gene may, for example, be Bmi1 gene, Mel18 gene, Ring1a/b gene, Phc1/2/3, Cbx2/4/6/7/8 gene, Eed gene, Ezh2 gene or Suz12 gene, preferably Bmi1 gene. Death of cells can be prevented by introduction of an apoptosis suppressor gene, such as BCL2 (B-cell lymphoma 2) gene or BCLXL (B-cell lymphoma-extra large) gene in the BCL2 family or Survivin, MCL1(myeloid cell leukemia1), preferably BCL2 gene or BCLXL gene. Suppression of expression of the tumor suppressor gene p53 is effective for inducing hematopoietic progenitor cells to differentiate into megakaryocytes .(see: Fuhrken etal., J. Biol. Chem., 283: 15589-15600 2008). Examples of tumor suppressor genes include p53 gene, p16 gene, p73 gene, Rb gene, BRCA1(breast cancer susceptibility gene 1) gene, BRCA2 gene and WT1 gene, and p53 gene is preferred. In addition, RNA genes which promote thrombopoiesis such as antisense RNAs, small interfering (si) RNAs, short hairpin (sh) RNAs, decoy RNA, ribozymes are also effective as target genes. These genes and RNAs include those having publicly known nucleotide sequences and their homologues obtained by homologous modification of these known sequences by conventional techniques. Hereinafter, genes introduced into hematopoietic progenitor cells for enhancement of their proliferative capability are referred to as target genes.

Target genes may be introduced into cells at any stage of differentiation from pluripotent stem cells to megakaryocytes, such as mesodermal cells, hematopoietic stem cells and hematopoietic progenitor cells. For introduction of target genes into these cells, techniques generally used for transfection of animal cells, for example, vector transfection using animal cell vectors of viral origin for gene therapy (see Verma, I.M., Nature, 389: 239, 1997 for vectors for gene therapy) such as retrovirus vectors represented by mouse stem cell virus (MSCV) and Molonry mouse leukemia virus (MmoLV), adenovirus vectors, adeno-associated vectors (AAVs), herpes simplex virus vectors, lentivirus vectors, Sendai virus vectors, the calcium phosphate coprecipitation method, the DEAE-Dextran method, the electroporation method, the liposome method, the lipofection method and the microinjection method, may be used. Among them, those which allow target genes to be integrated into the chromosomal DNA of the cells and expressed constitutively are preferred, and when viruses are used, retrovirus vectors, adeno-associated virus vectors or lentivirus vectors are preferred.

An adeno-associated virus (AAV) vector may be prepared, for example, as follows. First, 293 cells are transfected with a plasmid vector prepared by inserting a therapeutic gene between the ITRs (inverted terminal repeats) at both end of wild-type adeno-associated virus DNA and a helper plasmid for virus protein supplementation and subsequently with adenovirus as a helper virus or with a plasmid expressing an adenovirus helper genes to produce virus particles containing the AAV vector, which are used for transfection of hematopoietic progenitor cells. It is preferred to insert an appropriate promoter, enhancer, insulator or the like upstream of the target gene to regulate expression of the target gene. A marker gene such as a drug resistance gene may be introduced together with a target gene for easy selection of cells transfected with the target gene. The target gene may be a sense gene or an antisense gene.

A target gene may be introduced into cells via a mammalian expression vector or virus vector carrying the target gene functionally ligated downstream of an appropriate promoter so that the introduced target gene is expressed. Promoters such as CMV promoter, EF promoter and SV40 promoter may be used for constitutive expression of a target gene. A target gene may be functionally ligeted downstream of an endogeneous enhancer/promoter which induces gene expression at a certain stage of differentiation, such as glycoprotein IIb/IIIa enhance/promoter (see: Nat. Biotech 26, 209-211 (2008)) so that expression of the target gene is induced in the course of differentiation into megakaryocytes. Alternatively, an appropriate promoter may be placed under control of an element whose activity is regulated by a trans factor such as a drug-responsive element to provide a regulatory system which induces or suppresses expression of the target gene by addition of a drug or the like. Elements whose activity is controlled by a drug include, for example, tet operator element (see: Proc. Natl. Acad. Sci.USA 89, 5547-5551 (1992)), GAL4-dingin element (see: Proc. Natl. Acad. Sci.USA 91, 8180-8184 (1992)), Lac operator element (Environ. Mol. Mutagen. 28, 447-458 (1996)) and LexA operator element (see: the EMBO journal 7, 3975-3982 (1988)). Introduction of an appropriate gene having a ligand-binding domain, a DNA-binding domain and a transcriptional regulatory domain which activates or represses transcription responsive to a drug such as tetracycline, dexamthasone, tamoxyfen, estradiol, doxycycline or isopropyl-p-thiogalactopyranoside (IPTG) permits regulation of expression of a target gene downstream of the element by a drug. Transcriptional regulation by binding of a dimeric ligand containing a DNA-binding domain and a transcription regulatory domain is also possible, and it is possible to make such a dimer responsive to a certain drug by using the variable domain of an antibody, as disclosed in JP-A-2009-201504 . For regulated expression of a target gene, for example, the GeneSwitch™ system of Invitrogen, the LacSwitch II Inducible Mammalian Expression system of Agilent Technologies and the iDimerize™ system, the Tet-on™ system and the Tet-off™ system of Clontech may be used. Further, for a drug-responsive regulation of the amount of the protein expressed from a target gene, a target gene to be introduced may be fused with the destabilization domain of a mutant FK506 binding protein by using, for example, the ProtoTuner™ system of Clontech. In addition, it is possible to introduce a target gene at an appropriate location of a genome by using the homologous recombination technique (see: Nature 317, 230-234 (1985)). Further, an introduced target gene or an oncogene in a genome can be removed from the genome or repressed, for example, by using the Cre/Lox system or the FLP/frt system disclosed in Mammalian Genome 15, 677-685 (2004) singly or in combination. Further, removal of a target gene may be attained by directly introducing the Cre protein or the FLP protein at a certain stage of differentiation or by introducing a gene encoding such a protein. A target gene preliminarily introduced into cells can be removed at a certain stage of differentiation by expressing the Cre protein or the FLP protein under control of a drug-responsive element, as in the Cre-ER system disclosed in Developmental Biology 244, 305-318 (2002).

Hematopoietic progenitor cells with enhanced proliferative capability and/or differentiative capability by regulation of expression of a target gene by genetic manipulation may be used for production of megakaryocytes and platelets by the method of the present invention.

Since platelets are effective for preventing and alleviating a decrease in platelets due to leukemia, bone marrow transplantation and anticancer treatment, human platelets obtained by the method of the present invention can be stably supplied in the form of a platelet preparation. A platelet preparation can be prepared from platelets produced by the method of the present invention by recovering a fraction of a liquid culture rich in platelets released from magakaryocytes (for example, in the case of human platelets, an approximately 22- to 28 day culture of iPS cells) and separating platelets from other components by removing megakaryocytes and other blood cells by using a leukocyte reduction filter (available, for example, from TERUMO and Asahi Kasei Medical) or the like, or by precipitating non-platelet components by centrifugation at 100 to 150g for 10 to 15 minutes. A platelet preparation may contain other components which stabilize platelets in view of the storage instability of platelets. As conditions for platelet stabilization, a method well known to experts in this technical field may be selected. More specifically, platelets obtained (for example, washed platelets derived from human iPS cells) may be converted to a platelet preparation as follows.

ACD-A solution and FFP (fresh frozen plasma; prepared from whole blood obtained by blood donation, which contains all the other blood components other than blood cells such as albumin and a coagulation factor) are mixed at a ratio of 1:10, irradiated with 15-50 Gy radiation and stored with shaking at 20 to 24°C. The ACD-A solution is prepared by dissolving 22 g of sodium citrate/8 g of citric acid/22 g of glucose with water for injection to a total volume of 1 L.

When the above-mentioned method is used, the platelet density is set desirably at 1×10⁹ platelets/mL, for example.

Addition of GM6001(a broad-range hydroxamic acid-based metalloprotease inhibitor) (Calbiochem, La Jolla, CA, USA) prevents platelet deactivation during cryopreservation and storage at room temperature caused by cleavage of the molecules essential for platelet function such as GPIb-V-IX and GPVI. The present inventors confirmed that inactivation of platelets derived from mouse ES cells can be prevented by this method. For the mechanisms of inactivation of human platelets by this method, see Bergmeier, W et al., Cir Res 95: 677-683, 2004 and Gardiner, EE et al., J Thrombosis and Haemostasis, 5:1530-1537, 2007.

For a container of a preparation containing platelets, materials which activate platelets such as glass are preferably avoided.

Platelets produced by the method of the present invention may be used for treatment of diseases accompanied by a decrease in platelets and effective for treatment of various diseases.

As specific examples, lysosomal storage disease such as Gaucher's disease and mucopolysaccharidosis, adrenoleukodystrophy, various kinds of cancers and tumors, especially blood cancers such as acute or chronic leukemia, Fanconi syndrome, aplastic anemia, granulocytopenia, lymphopenia, thrombocytopenia, idiopathic thrombocytopenic purpura, thrombotic thrombocytopenic purpura, Kasabach-Merritt syndrome, malignant lymphoma, Hodgkin's disease, multiple myeloma, chronic hepatopathy, renal failure, massive blood transfusion of bank blood or during operation, hepatitis B, hepatitis C, severe infections, systemic lupus erythematodes, articular rheumatism, xerodermosteosis, systemic sclerosis, polymyositis, dermatomyositis, mixed connective tissue disease, polyarteritis nodosa, Hashimoto's disease, Basedow's disease, myasthenia gravis, insulin dependent diabetes mellitus, autoimmune hemolytic anemia, snake bite, hemolytic uremic syndrome, hypersplenism, bleeding, Bernard-Soulier syndrome, Glanzmann's thrombasthenia, uremia, myelodysplastic syndrome, polycythemia rubra vera, erythremia, myeloproliferative disease, and the like may be mentioned. Now, the specific compound to be used in the present invention will be described in terms of the definitions of terms used for it and its best mode.

In the compound to be used in the present invention, "n" denotes normal, "i" denotes iso, "s" denotes secondary, "t" denotes tertiary, "c" denotes cyclo, "o" denotes ortho, "m" denotes meta, and "p" denotes para.

First, the terms in the respective substituents R¹ to R²⁰ and V¹ to V⁴ will be explained.

As a halogen atom, a fluorine atom, a chlorine atom, a bromine atom or an iodine atom may be mentioned.

A C₁₋₃ alkyl group may be linear, branched or a C₃ cycloalkyl group, and methyl, ethyl, n-propyl, i-propyl and c-propyl and the like may be mentioned.

A C₁₋₃ alkoxy group may be linear, branched or a C₃ cycloalkoxy group, and as specific examples, methoxy, ethoxy, n-propoxy, i-propoxy, c-propoxy or the like may be mentioned.

Herein, the expression "may be substituted" means that a group may have substituents in any positions of a group in each of which a substituent may be present, and that each substituent is dependent of one another.

For example, the expression "a C₁₋₃ alkoxy group which may be substituted with one or more halogen atoms" means an unsubstituted C₁₋₃ alkoxy group or an alkoxy group with a C₁₋₃ alkyl group in which optional hydrogen atom(s) may be substituted with halogen atom(s) provided that the number of halogen atoms are 2 or more, each halogen atoms may be identical to or different from one another, such as a trifluoromethoxy group, a 2,2,2-trifluoroethoxy group or a 1,1-difluoroethoxy group.

The wavy line in the formula of a group indicates a "site of bonding".

Preferred examples of the substituents in the compounds of the present invention represented by the formula (I) are given below. 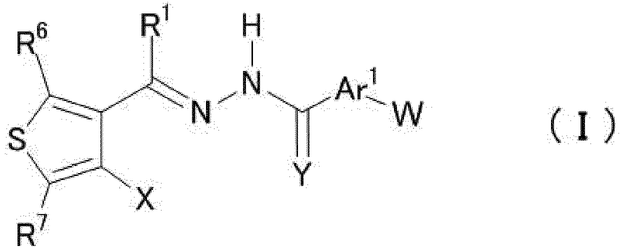 W is a substituent represented by the formula (Ia):

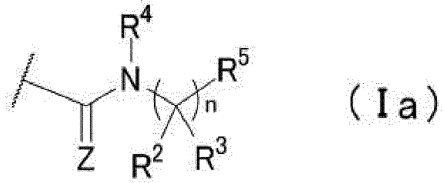

R¹ is a methyl group.

R², R³, R⁴ and R⁶ are a hydrogen atom.

Preferred examples of R⁵ are a phenyl group which may be substituted with one or more substituents independently represented by V¹.

As V¹, the formulae (V), (VI), (VII), (VIII), (IX), (X), (XI), (XII), (XIII), (XIV), (XV), (XVI), (XVII), (XVIII), (XIX), (XX), (XXI) and (XXII) may be mentioned.

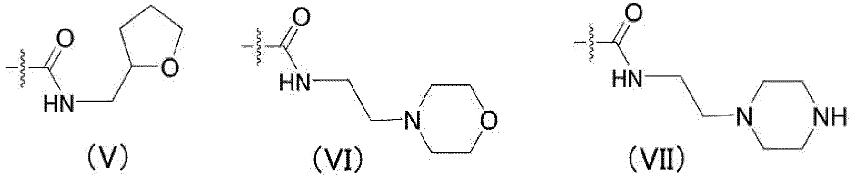

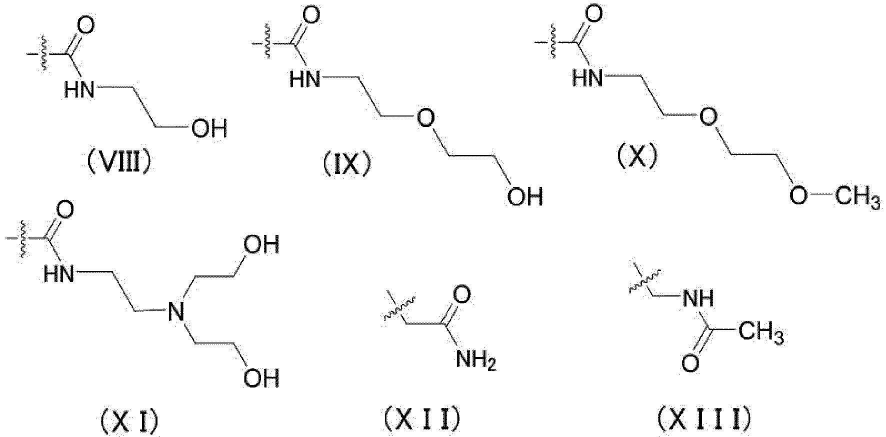

R⁵ is a phenyl group, a 2-pyridyl group, a 3-pyridyl group, a 4-pyridyl group, a 3-pyridazinyl group, a 4-pyridazinyl group, a 2-pyrimidinyl group, a 4-pyrimidinyl group, a 5-pyrimidinyl group, a 2-pyrazinyl group and groups obtained by substituting these groups with one or more substituents selected from the formulae (V), (VI), (VII), (VIII), (IX), (X), (XI), (XII), (XIII), (XIV), (XV), (XVI), (XVII), (XVIII), (XIX), (XX), (XXI) and (XXII).

Particularly preferred examples of R⁵ are a 4-pyridyl group, a phenyl group (the phenyl group is unsubstituted or substituted with one or more substituents selected from the formulae (V), (VI), (VII), (VIII), (IX), (X), (XI), (XII), (XIII), (XIV), (XV), (XVI), (XVII), (XVIII), (XIX), (XX), (XXI) and (XXII)) and the like.

The most preferred examples of R⁵ are a 4-pyridyl group and a phenyl group substituted with one or more substituents selected from the formulae (VII), (VIII), (XI) and (XII).

R⁷ is a phenyl group (the phenyl group is substituted with one or more substituents selected from the group consisting of methyl groups, t-butyl groups, halogen atoms, methoxy groups, trifluoromethyl groups and trifluoromethoxy groups, or is represented by any one of formulae (A01), (A02), (A03), (A04), (A05), (A06), (A07), (A08), (A09), (A10), (A11), (A12), (A13), (A14) and (A15)).

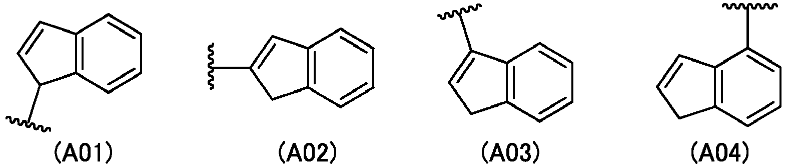

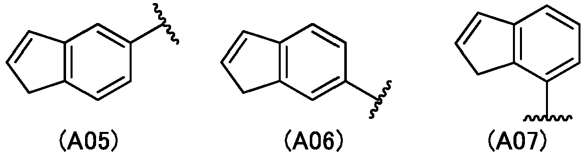

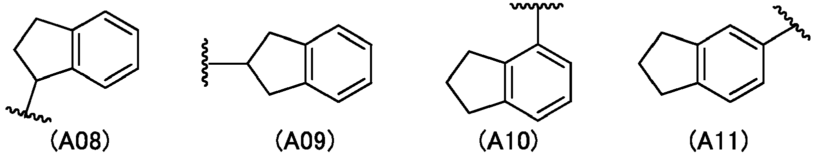

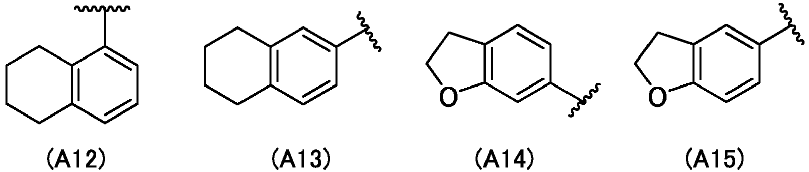

Particularly preferred examples of R⁷ are a phenyl group (the phenyl group is substituted with one or more substituents selected from the group consisting of methyl groups, t-butyl groups, halogen atoms, methoxy groups, trifluoromethyl groups and trifluoromethoxy groups) and the formulae (A05), (A06), (A08), (A09), (A10), (A11), (A12), (A13), (A14) and (A15).

More specific particular preferred examples are a phenyl group (the phenyl group is substituted with one or more substituents selected from the group consisting of methyl groups, t-butyl groups, halogen atoms, methoxy groups, trifluoromethyl groups and trifluoromethoxy groups) and the formulae (A11), (A13) and (A15).

Ar¹ is represented by the formula (IV).

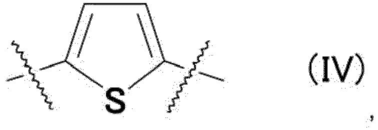

X is OH.

A preferred example of Y is an oxygen atom.

A preferred example of Z is an oxygen atom.

n is an integer of 1 or 2, more preferably an integer of 1. When n is 1, it is particularly preferred that R⁵ is a 4-pyridyl group or a phenyl group substituted with one or more substituents selected from the formulae (VII), (VIII), (XI) and (XII).

Preferred examples of the compounds of the present invention are compounds wherein Ra, Ar and Q are any of the following combinations shown in Tables 1 to 13, tautomers or pharmaceutically acceptable salts of the compounds or solvates thereof. The symbols in Tables 1 to 13 denote the following substituents. 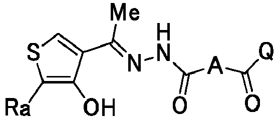 Compounds wherein A = A², A³, A⁴, or A⁵, or wherein Q = Q², Q⁶, Q⁹, Q¹², Q¹³, or Q¹⁷ are comparative compounds.

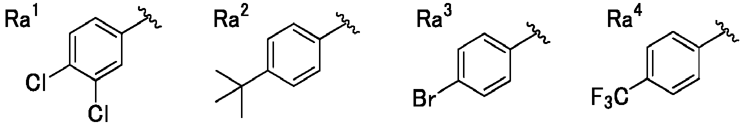

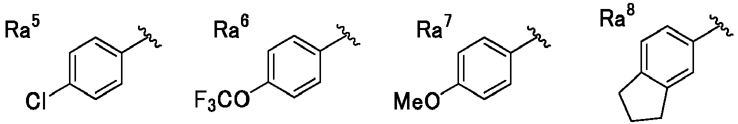

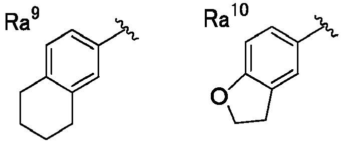

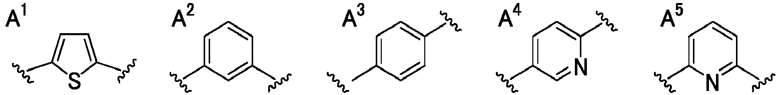

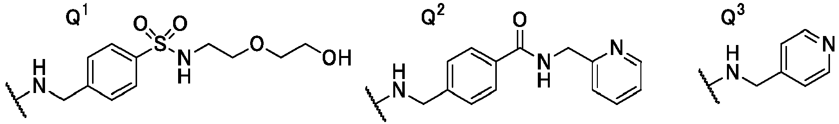

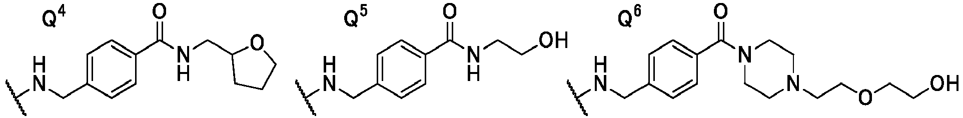

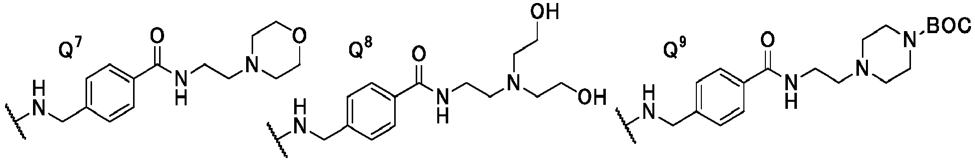

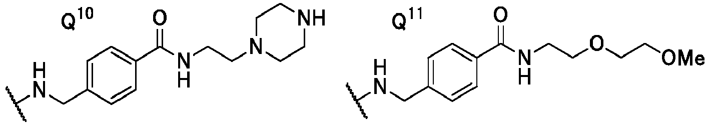

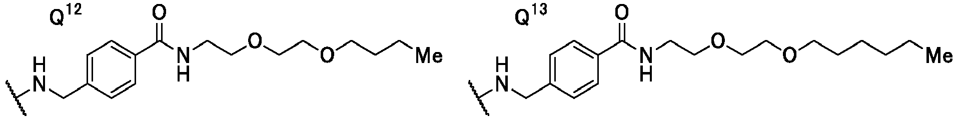

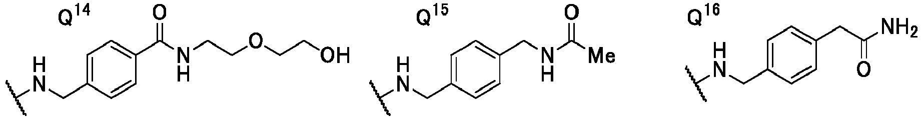

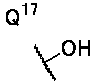

**TABLE 1**

| No. | Ra | A | Q | No. | Ra | A | Q |
|---|---|---|---|---|---|---|---|
| 1 | Ra¹ | A¹ | Q¹ | 35 | Ra¹ | A³ | Q¹ |
| 2 | Ra¹ | A¹ | Q² | 36 | Ra¹ | A³ | Q² |
| 3 | Ra¹ | A¹ | Q³ | 37 | Ra¹ | A³ | Q³ |
| 4 | Ra¹ | A¹ | Q⁴ | 38 | Ra¹ | A³ | Q⁴ |
| 5 | Ra¹ | A¹ | Q⁵ | 39 | Ra¹ | A³ | Q⁵ |
| 6 | Ra¹ | A¹ | Q⁶ | 40 | Ra¹ | A³ | Q⁶ |
| 7 | Ra¹ | A¹ | Q⁷ | 41 | Ra¹ | A³ | Q⁷ |
| 8 | Ra¹ | A¹ | Q⁸ | 42 | Ra¹ | A³ | Q⁸ |
| 9 | Ra¹ | A¹ | Q⁹ | 43 | Ra¹ | A³ | Q⁹ |
| 10 | Ra¹ | A¹ | Q¹⁰ | 44 | Ra¹ | A³ | Q¹⁰ |
| 11 | Ra¹ | A¹ | Q¹¹ | 45 | Ra¹ | A³ | Q¹¹ |
| 12 | Ra¹ | A¹ | Q¹² | 46 | Ra¹ | A³ | Q¹² |
| 13 | Ra¹ | A¹ | Q¹³ | 47 | Ra¹ | A³ | Q¹³ |
| 14 | Ra¹ | A¹ | Q¹⁴ | 48 | Ra¹ | A³ | Q¹⁴ |
| 15 | Ra¹ | A¹ | Q¹⁵ | 49 | Ra¹ | A³ | Q¹⁵ |
| 16 | Ra¹ | A¹ | Q¹⁶ | 50 | Ra¹ | A³ | Q¹⁶ |
| 17 | Ra¹ | A¹ | Q¹⁷ | 51 | Ra¹ | A³ | Q¹⁷ |
| 18 | Ra¹ | A² | Q¹ | 52 | Ra¹ | A⁴ | Q¹ |
| 19 | Ra¹ | A² | Q² | 53 | Ra¹ | A⁴ | Q² |
| 20 | Ra¹ | A² | Q³ | 54 | Ra¹ | A⁴ | Q³ |
| 21 | Ra¹ | A² | Q⁴ | 55 | Ra¹ | A⁴ | Q⁴ |
| 22 | Ra¹ | A² | Q⁵ | 56 | Ra¹ | A⁴ | Q⁵ |
| 23 | Ra¹ | A² | Q⁶ | 57 | Ra¹ | A⁴ | Q⁶ |
| 24 | Ra¹ | A² | Q⁷ | 58 | Ra¹ | A⁴ | Q⁷ |
| 25 | Ra¹ | A² | Q⁸ | 59 | Ra¹ | A⁴ | Q⁸ |
| 26 | Ra¹ | A² | Q⁹ | 60 | Ra¹ | A⁴ | Q⁹ |
| 27 | Ra¹ | A² | Q¹⁰ | 61 | Ra¹ | A⁴ | Q¹⁰ |
| 28 | Ra¹ | A² | Q¹¹ | 62 | Ra¹ | A⁴ | Q¹¹ |
| 29 | Ra¹ | A² | Q¹² | 63 | Ra¹ | A⁴ | Q¹² |
| 30 | Ra¹ | A² | Q¹³ | 64 | Ra¹ | A⁴ | Q¹³ |
| 31 | Ra¹ | A² | Q¹⁴ | 65 | Ra¹ | A⁴ | Q¹⁴ |
| 32 | Ra¹ | A² | Q¹⁵ | 66 | Ra¹ | A⁴ | Q¹⁵ |
| 33 | Ra¹ | A² | Q¹⁶ | 67 | Ra¹ | A⁴ | Q¹⁶ |
| 34 | Ra¹ | A² | Q¹⁷ | 68 | Ra¹ | A⁴ | Q¹⁷ |

**TABLE 2**

| No. | Ra | A | Q | No. | Ra | A | Q |
|---|---|---|---|---|---|---|---|
| 69 | Ra¹ | A⁵ | Q¹ | 103 | Ra² | A² | Q¹ |
| 70 | Ra¹ | A⁵ | Q² | 104 | Ra² | A² | Q² |
| 71 | Ra¹ | A⁵ | Q³ | 105 | Ra² | A² | Q³ |
| 72 | Ra¹ | A⁵ | Q⁴ | 106 | Ra² | A² | Q⁴ |
| 73 | Ra¹ | A⁵ | Q⁵ | 107 | Ra² | A² | Q⁵ |
| 74 | Ra¹ | A⁵ | Q⁶ | 108 | Ra² | A² | Q⁶ |
| 75 | Ra¹ | A⁵ | Q⁷ | 109 | Ra² | A² | Q⁷ |
| 76 | Ra¹ | A⁵ | Q⁸ | 110 | Ra² | A² | Q⁸ |
| 77 | Ra¹ | A⁵ | Q⁹ | 111 | Ra² | A² | Q⁹ |
| 78 | Ra¹ | A⁵ | Q¹⁰ | 112 | Ra² | A² | Q¹⁰ |
| 79 | Ra¹ | A⁵ | Q¹¹ | 113 | Ra² | A² | Q¹¹ |
| 80 | Ra¹ | A⁵ | Q¹² | 114 | Ra² | A² | Q¹² |
| 81 | Ra¹ | A⁵ | Q¹³ | 115 | Ra² | A² | Q¹³ |
| 82 | Ra¹ | A⁵ | Q¹⁴ | 116 | Ra² | A² | Q¹⁴ |
| 83 | Ra¹ | A⁵ | Q¹⁵ | 117 | Ra² | A² | Q¹⁵ |
| 84 | Ra¹ | A⁵ | Q¹⁶ | 118 | Ra² | A² | Q¹⁶ |
| 85 | Ra¹ | A⁵ | Q¹⁷ | 119 | Ra² | A² | Q¹⁷ |
| 86 | Ra² | A¹ | Q¹ | 120 | Ra² | A³ | Q¹ |
| 87 | Ra² | A¹ | Q² | 121 | Ra² | A³ | Q² |
| 88 | Ra² | A¹ | Q³ | 122 | Ra² | A³ | Q³ |
| 89 | Ra² | A¹ | Q⁴ | 123 | Ra² | A³ | Q⁴ |
| 90 | Ra² | A¹ | Q⁵ | 124 | Ra² | A³ | Q⁵ |
| 91 | Ra² | A¹ | Q⁶ | 125 | Ra² | A³ | Q⁶ |
| 92 | Ra² | A¹ | Q⁷ | 126 | Ra² | A³ | Q⁷ |
| 93 | Ra² | A¹ | Q⁸ | 127 | Ra² | A³ | Q⁸ |
| 94 | Ra² | A¹ | Q⁹ | 128 | Ra² | A³ | Q⁹ |
| 95 | Ra² | A¹ | Q¹⁰ | 129 | Ra² | A³ | Q¹⁰ |
| 96 | Ra² | A¹ | Q¹¹ | 130 | Ra² | A³ | Q¹¹ |
| 97 | Ra² | A¹ | Q¹² | 131 | Ra² | A³ | Q¹² |
| 98 | Ra² | A¹ | Q¹³ | 132 | Ra² | A³ | Q¹³ |
| 99 | Ra² | A¹ | Q¹⁴ | 133 | Ra² | A³ | Q¹⁴ |
| 100 | Ra² | A¹ | Q¹⁵ | 134 | Ra² | A³ | Q¹⁵ |
| 101 | Ra² | A¹ | Q¹⁶ | 135 | Ra² | A³ | Q¹⁶ |
| 102 | Ra² | A¹ | Q¹⁷ | 136 | Ra² | A³ | Q¹⁷ |

**TABLE 3**

| No. | Ra | A | Q | No. | Ra | A | Q |
|---|---|---|---|---|---|---|---|
| 137 | Ra² | A⁴ | Q¹ | 171 | Ra³ | A¹ | Q¹ |
| 138 | Ra² | A⁴ | Q² | 172 | Ra³ | A¹ | Q² |
| 139 | Ra² | A⁴ | Q³ | 173 | Ra³ | A¹ | Q³ |
| 140 | Ra² | A⁴ | Q⁴ | 174 | Ra³ | A¹ | Q⁴ |
| 141 | Ra² | A⁴ | Q⁵ | 175 | Ra³ | A¹ | Q⁵ |
| 142 | Ra² | A⁴ | Q⁶ | 176 | Ra³ | A¹ | Q⁶ |
| 143 | Ra² | A⁴ | Q⁷ | 177 | Ra³ | A¹ | Q⁷ |
| 144 | Ra² | A⁴ | Q⁸ | 178 | Ra³ | A¹ | Q⁸ |
| 145 | Ra² | A⁴ | Q⁹ | 179 | Ra³ | A¹ | Q⁹ |
| 146 | Ra² | A⁴ | Q¹⁰ | 180 | Ra³ | A¹ | Q¹⁰ |
| 147 | Ra² | A⁴ | Q¹¹ | 181 | Ra³ | A¹ | Q¹¹ |
| 148 | Ra² | A⁴ | Q¹² | 182 | Ra³ | A¹ | Q¹² |
| 149 | Ra² | A⁴ | Q¹³ | 183 | Ra³ | A¹ | Q¹³ |
| 150 | Ra² | A⁴ | Q¹⁴ | 184 | Ra³ | A¹ | Q¹⁴ |
| 151 | Ra² | A⁴ | Q¹⁵ | 185 | Ra³ | A¹ | Q¹⁵ |
| 152 | Ra² | A⁴ | Q¹⁶ | 186 | Ra³ | A¹ | Q¹⁶ |
| 153 | Ra² | A⁴ | Q¹⁷ | 187 | Ra³ | A¹ | Q¹⁷ |
| 154 | Ra² | A⁵ | Q¹ | 188 | Ra³ | A² | Q¹ |
| 155 | Ra² | A⁵ | Q² | 189 | Ra³ | A² | Q² |
| 156 | Ra² | A⁵ | Q³ | 190 | Ra³ | A² | Q³ |
| 157 | Ra² | A⁵ | Q⁴ | 191 | Ra³ | A² | Q⁴ |
| 158 | Ra² | A⁵ | Q⁵ | 192 | Ra³ | A² | Q⁵ |
| 159 | Ra² | A⁵ | Q⁶ | 193 | Ra³ | A² | Q⁶ |
| 160 | Ra² | A⁵ | Q⁷ | 194 | Ra³ | A² | Q⁷ |
| 161 | Ra² | A⁵ | Q⁸ | 195 | Ra³ | A² | Q⁸ |
| 162 | Ra² | A⁵ | Q⁹ | 196 | Ra³ | A² | Q⁹ |
| 163 | Ra² | A⁵ | Q¹⁰ | 197 | Ra³ | A² | Q¹⁰ |
| 164 | Ra² | A⁵ | Q¹¹ | 198 | Ra³ | A² | Q¹¹ |
| 165 | Ra² | A⁵ | Q¹² | 199 | Ra³ | A² | Q¹² |
| 166 | Ra² | A⁵ | Q¹³ | 200 | Ra³ | A² | Q¹³ |
| 167 | Ra² | A⁵ | Q¹⁴ | 201 | Ra³ | A² | Q¹⁴ |
| 168 | Ra² | A⁵ | Q¹⁵ | 202 | Ra³ | A² | Q¹⁵ |
| 169 | Ra² | A⁵ | Q¹⁶ | 203 | Ra³ | A² | Q¹⁶ |
| 170 | Ra² | A⁵ | Q¹⁷ | 204 | Ra³ | A² | Q¹⁷ |

**TABLE 4**

| No. | Ra | A | Q | No. | Ra | A | Q |
|---|---|---|---|---|---|---|---|
| 205 | Ra³ | A³ | Q¹ | 239 | Ra³ | A⁵ | Q¹ |
| 206 | Ra³ | A³ | Q² | 240 | Ra³ | A⁵ | Q² |
| 207 | Ra³ | A³ | Q³ | 241 | Ra³ | A⁵ | Q³ |
| 208 | Ra³ | A³ | Q⁴ | 242 | Ra³ | A⁵ | Q⁴ |
| 209 | Ra³ | A³ | Q⁵ | 243 | Ra³ | A⁵ | Q⁵ |
| 210 | Ra³ | A³ | Q⁶ | 244 | Ra³ | A⁵ | Q⁶ |
| 211 | Ra³ | A³ | Q⁷ | 245 | Ra³ | A⁵ | Q⁷ |
| 212 | Ra³ | A³ | Q⁸ | 246 | Ra³ | A⁵ | Q⁸ |
| 213 | Ra³ | A³ | Q⁹ | 247 | Ra³ | A⁵ | Q⁹ |
| 214 | Ra³ | A³ | Q¹⁰ | 248 | Ra³ | A⁵ | Q¹⁰ |
| 215 | Ra³ | A³ | Q¹¹ | 249 | Ra³ | A⁵ | Q¹¹ |
| 216 | Ra³ | A³ | Q¹² | 250 | Ra³ | A⁵ | Q¹² |
| 217 | Ra³ | A³ | Q¹³ | 251 | Ra³ | A⁵ | Q¹³ |
| 218 | Ra³ | A³ | Q¹⁴ | 252 | Ra³ | A⁵ | Q¹⁴ |
| 219 | Ra³ | A³ | Q¹⁵ | 253 | Ra³ | A⁵ | Q¹⁵ |
| 220 | Ra³ | A³ | Q¹⁶ | 254 | Ra³ | A⁵ | Q¹⁶ |
| 221 | Ra³ | A³ | Q¹⁷ | 255 | Ra³ | A⁵ | Q¹⁷ |
| 222 | Ra³ | A⁴ | Q¹ | 256 | Ra⁴ | A¹ | Q¹ |
| 223 | Ra³ | A⁴ | Q² | 257 | Ra⁴ | A¹ | Q² |
| 224 | Ra³ | A⁴ | Q³ | 258 | Ra⁴ | A¹ | Q³ |
| 225 | Ra³ | A⁴ | Q⁴ | 259 | Ra⁴ | A¹ | Q⁴ |
| 226 | Ra³ | A⁴ | Q⁵ | 260 | Ra⁴ | A¹ | Q⁵ |
| 227 | Ra³ | A⁴ | Q⁶ | 261 | Ra⁴ | A¹ | Q⁶ |
| 228 | Ra³ | A⁴ | Q⁷ | 262 | Ra⁴ | A¹ | Q⁷ |
| 229 | Ra³ | A⁴ | Q⁸ | 263 | Ra⁴ | A¹ | Q⁸ |
| 230 | Ra³ | A⁴ | Q⁹ | 264 | Ra⁴ | A¹ | Q⁹ |
| 231 | Ra³ | A⁴ | Q¹⁰ | 265 | Ra⁴ | A¹ | Q¹⁰ |
| 232 | Ra³ | A⁴ | Q¹¹ | 266 | Ra⁴ | A¹ | Q¹¹ |
| 233 | Ra³ | A⁴ | Q¹² | 267 | Ra⁴ | A¹ | Q¹² |
| 234 | Ra³ | A⁴ | Q¹³ | 268 | Ra⁴ | A¹ | Q¹³ |
| 235 | Ra³ | A⁴ | Q¹⁴ | 269 | Ra⁴ | A¹ | Q¹⁴ |
| 236 | Ra³ | A⁴ | Q¹⁵ | 270 | Ra⁴ | A¹ | Q¹⁵ |
| 237 | Ra³ | A⁴ | Q¹⁶ | 271 | Ra⁴ | A¹ | Q¹⁶ |
| 238 | Ra³ | A⁴ | Q¹⁷ | 272 | Ra⁴ | A¹ | Q¹⁷ |

**TABLE 5**

| No. | Ra | A | Q | No. | Ra | A | Q |
|---|---|---|---|---|---|---|---|
| 273 | Ra⁴ | A² | Q¹ | 307 | Ra⁴ | A⁴ | Q¹ |
| 274 | Ra⁴ | A² | Q² | 308 | Ra⁴ | A⁴ | Q² |
| 275 | Ra⁴ | A² | Q³ | 309 | Ra⁴ | A⁴ | Q³ |
| 276 | Ra⁴ | A² | Q⁴ | 310 | Ra⁴ | A⁴ | Q⁴ |
| 277 | Ra⁴ | A² | Q⁵ | 311 | Ra⁴ | A⁴ | Q⁵ |
| 278 | Ra⁴ | A² | Q⁶ | 312 | Ra⁴ | A⁴ | Q⁶ |
| 279 | Ra⁴ | A² | Q⁷ | 313 | Ra⁴ | A⁴ | Q⁷ |
| 280 | Ra⁴ | A² | Q⁸ | 314 | Ra⁴ | A⁴ | Q⁸ |
| 281 | Ra⁴ | A² | Q⁹ | 315 | Ra⁴ | A⁴ | Q⁹ |
| 282 | Ra⁴ | A² | Q¹⁰ | 316 | Ra⁴ | A⁴ | Q¹⁰ |
| 283 | Ra⁴ | A² | Q¹¹ | 317 | Ra⁴ | A⁴ | Q¹¹ |
| 284 | Ra⁴ | A² | Q¹² | 318 | Ra⁴ | A⁴ | Q¹² |
| 285 | Ra⁴ | A² | Q¹³ | 319 | Ra⁴ | A⁴ | Q¹³ |
| 286 | Ra⁴ | A² | Q¹⁴ | 320 | Ra⁴ | A⁴ | Q¹⁴ |
| 287 | Ra⁴ | A² | Q¹⁵ | 321 | Ra⁴ | A⁴ | Q¹⁵ |
| 288 | Ra⁴ | A² | Q¹⁶ | 322 | Ra⁴ | A⁴ | Q¹⁶ |
| 289 | Ra⁴ | A² | Q¹⁷ | 323 | Ra⁴ | A⁴ | Q¹⁷ |
| 290 | Ra⁴ | A³ | Q¹ | 324 | Ra⁴ | A⁵ | Q¹ |
| 291 | Ra⁴ | A³ | Q² | 325 | Ra⁴ | A⁵ | Q² |
| 292 | Ra⁴ | A³ | Q³ | 326 | Ra⁴ | A⁵ | Q³ |
| 293 | Ra⁴ | A³ | Q⁴ | 327 | Ra⁴ | A⁵ | Q⁴ |
| 294 | Ra⁴ | A³ | Q⁵ | 328 | Ra⁴ | A⁵ | Q⁵ |
| 295 | Ra⁴ | A³ | Q⁶ | 329 | Ra⁴ | A⁵ | Q⁶ |
| 296 | Ra⁴ | A³ | Q⁷ | 330 | Ra⁴ | A⁵ | Q⁷ |
| 297 | Ra⁴ | A³ | Q⁸ | 331 | Ra⁴ | A⁵ | Q⁸ |
| 298 | Ra⁴ | A³ | Q⁹ | 332 | Ra⁴ | A⁵ | Q⁹ |
| 299 | Ra⁴ | A³ | Q¹⁰ | 333 | Ra⁴ | A⁵ | Q¹⁰ |
| 300 | Ra⁴ | A³ | Q¹¹ | 334 | Ra⁴ | A⁵ | Q¹¹ |
| 301 | Ra⁴ | A³ | Q¹² | 335 | Ra⁴ | A⁵ | Q¹² |
| 302 | Ra⁴ | A³ | Q¹³ | 336 | Ra⁴ | A⁵ | Q¹³ |
| 303 | Ra⁴ | A³ | Q¹⁴ | 337 | Ra⁴ | A⁵ | Q¹⁴ |
| 304 | Ra⁴ | A³ | Q¹⁵ | 338 | Ra⁴ | A⁵ | Q¹⁵ |
| 305 | Ra⁴ | A³ | Q¹⁶ | 339 | Ra⁴ | A⁵ | Q¹⁶ |
| 306 | Ra⁴ | A³ | Q¹⁷ | 340 | Ra⁴ | A⁵ | Q¹⁷ |

**TABLE 6**

| No. | Ra | A | Q | No. | Ra | A | Q |
|---|---|---|---|---|---|---|---|
| 341 | Ra⁵ | A¹ | Q¹ | 375 | Ra⁵ | A³ | Q¹ |
| 342 | Ra⁵ | A¹ | Q² | 376 | Ra⁵ | A³ | Q² |
| 343 | Ra⁵ | A¹ | Q³ | 377 | Ra⁵ | A³ | Q³ |
| 344 | Ra⁵ | A¹ | Q⁴ | 378 | Ra⁵ | A³ | Q⁴ |
| 345 | Ra⁵ | A¹ | Q⁵ | 379 | Ra⁵ | A³ | Q⁵ |
| 346 | Ra⁵ | A¹ | Q⁶ | 380 | Ra⁵ | A³ | Q⁶ |
| 347 | Ra⁵ | A¹ | Q⁷ | 381 | Ra⁵ | A³ | Q⁷ |
| 348 | Ra⁵ | A¹ | Q⁸ | 382 | Ra⁵ | A³ | Q⁸ |
| 349 | Ra⁵ | A¹ | Q⁹ | 383 | Ra⁵ | A³ | Q⁹ |
| 350 | Ra⁵ | A¹ | Q¹⁰ | 384 | Ra⁵ | A³ | Q¹⁰ |
| 351 | Ra⁵ | A¹ | Q¹¹ | 385 | Ra⁵ | A³ | Q¹¹ |
| 352 | Ra⁵ | A¹ | Q¹² | 386 | Ra⁵ | A³ | Q¹² |
| 353 | Ra⁵ | A¹ | Q¹³ | 387 | Ra⁵ | A³ | Q¹³ |
| 354 | Ra⁵ | A¹ | Q¹⁴ | 388 | Ra⁵ | A³ | Q¹⁴ |
| 355 | Ra⁵ | A¹ | Q¹⁵ | 389 | Ra⁵ | A³ | Q¹⁵ |
| 356 | Ra⁵ | A¹ | Q¹⁶ | 390 | Ra⁵ | A³ | Q¹⁶ |
| 357 | Ra⁵ | A¹ | Q¹⁷ | 391 | Ra⁵ | A³ | Q¹⁷ |
| 358 | Ra⁵ | A² | Q¹ | 392 | Ra⁵ | A⁴ | Q¹ |
| 359 | Ra⁵ | A² | Q² | 393 | Ra⁵ | A⁴ | Q² |
| 360 | Ra⁵ | A² | Q³ | 394 | Ra⁵ | A⁴ | Q³ |
| 361 | Ra⁵ | A² | Q⁴ | 395 | Ra⁵ | A⁴ | Q⁴ |
| 362 | Ra⁵ | A² | Q⁵ | 396 | Ra⁵ | A⁴ | Q⁵ |
| 363 | Ra⁵ | A² | Q⁶ | 397 | Ra⁵ | A⁴ | Q⁶ |
| 364 | Ra⁵ | A² | Q⁷ | 398 | Ra⁵ | A⁴ | Q⁷ |
| 365 | Ra⁵ | A² | Q⁸ | 399 | Ra⁵ | A⁴ | Q⁸ |
| 366 | Ra⁵ | A² | Q⁹ | 400 | Ra⁵ | A⁴ | Q⁹ |
| 367 | Ra⁵ | A² | Q¹⁰ | 401 | Ra⁵ | A⁴ | Q¹⁰ |
| 368 | Ra⁵ | A² | Q¹¹ | 402 | Ra⁵ | A⁴ | Q¹¹ |
| 369 | Ra⁵ | A² | Q¹² | 403 | Ra⁵ | A⁴ | Q¹² |
| 370 | Ra⁵ | A² | Q¹³ | 404 | Ra⁵ | A⁴ | Q¹³ |
| 371 | Ra⁵ | A² | Q¹⁴ | 405 | Ra⁵ | A⁴ | Q¹⁴ |
| 372 | Ra⁵ | A² | Q¹⁵ | 406 | Ra⁵ | A⁴ | Q¹⁵ |
| 373 | Ra⁵ | A² | Q¹⁶ | 407 | Ra⁵ | A⁴ | Q¹⁶ |
| 374 | Ra⁵ | A² | Q¹⁷ | 408 | Ra⁵ | A⁴ | Q¹⁷ |

**TABLE 7**

| No. | Ra | A | Q | No. | Ra | A | Q |
|---|---|---|---|---|---|---|---|
| 409 | Ra⁵ | A⁵ | Q¹ | 443 | Ra⁶ | A² | Q¹ |
| 410 | Ra⁵ | A⁵ | Q² | 444 | Ra⁶ | A² | Q² |
| 411 | Ra⁵ | A⁵ | Q³ | 445 | Ra⁶ | A² | Q³ |
| 412 | Ra⁵ | A⁵ | Q⁴ | 446 | Ra⁶ | A² | Q⁴ |
| 413 | Ra⁵ | A⁵ | Q⁵ | 447 | Ra⁶ | A² | Q⁵ |
| 414 | Ra⁵ | A⁵ | Q⁶ | 448 | Ra⁶ | A² | Q⁶ |
| 415 | Ra⁵ | A⁵ | Q⁷ | 449 | Ra⁶ | A² | Q⁷ |
| 416 | Ra⁵ | A⁵ | Q⁸ | 450 | Ra⁶ | A² | Q⁸ |
| 417 | Ra⁵ | A⁵ | Q⁹ | 451 | Ra⁶ | A² | Q⁹ |
| 418 | Ra⁵ | A⁵ | Q¹⁰ | 452 | Ra⁶ | A² | Q¹⁰ |
| 419 | Ra⁵ | A⁵ | Q¹¹ | 453 | Ra⁶ | A² | Q¹¹ |
| 420 | Ra⁵ | A⁵ | Q¹² | 454 | Ra⁶ | A² | Q¹² |
| 421 | Ra⁵ | A⁵ | Q¹³ | 455 | Ra⁶ | A² | Q¹³ |
| 422 | Ra⁵ | A⁵ | Q¹⁴ | 456 | Ra⁶ | A² | Q¹⁴ |
| 423 | Ra⁵ | A⁵ | Q¹⁵ | 457 | Ra⁶ | A² | Q¹⁵ |
| 424 | Ra⁵ | A⁵ | Q¹⁶ | 458 | Ra⁶ | A² | Q¹⁶ |
| 425 | Ra⁵ | A⁵ | Q¹⁷ | 459 | Ra⁶ | A² | Q¹⁷ |
| 426 | Ra⁶ | A¹ | Q¹ | 460 | Ra⁶ | A³ | Q¹ |
| 427 | Ra⁶ | A¹ | Q² | 461 | Ra⁶ | A³ | Q² |
| 428 | Ra⁶ | A¹ | Q³ | 462 | Ra⁶ | A³ | Q³ |
| 429 | Ra⁶ | A¹ | Q⁴ | 463 | Ra⁶ | A³ | Q⁴ |
| 430 | Ra⁶ | A¹ | Q⁵ | 464 | Ra⁶ | A³ | Q⁵ |
| 431 | Ra⁶ | A¹ | Q⁶ | 465 | Ra⁶ | A³ | Q⁶ |
| 432 | Ra⁶ | A¹ | Q⁷ | 466 | Ra⁶ | A³ | Q⁷ |
| 433 | Ra⁶ | A¹ | Q⁸ | 467 | Ra⁶ | A³ | Q⁸ |
| 434 | Ra⁶ | A¹ | Q⁹ | 468 | Ra⁶ | A³ | Q⁹ |
| 435 | Ra⁶ | A¹ | Q¹⁰ | 469 | Ra⁶ | A³ | Q¹⁰ |
| 436 | Ra⁶ | A¹ | Q¹¹ | 470 | Ra⁶ | A³ | Q¹¹ |
| 437 | Ra⁶ | A¹ | Q¹² | 471 | Ra⁶ | A³ | Q¹² |
| 438 | Ra⁶ | A¹ | Q¹³ | 472 | Ra⁶ | A³ | Q¹³ |
| 439 | Ra⁶ | A¹ | Q¹⁴ | 473 | Ra⁶ | A³ | Q¹⁴ |
| 440 | Ra⁶ | A¹ | Q¹⁵ | 474 | Ra⁶ | A³ | Q¹⁵ |
| 441 | Ra⁶ | A¹ | Q¹⁶ | 475 | Ra⁶ | A³ | Q¹⁶ |
| 442 | Ra⁶ | A¹ | Q¹⁷ | 476 | Ra⁶ | A³ | Q¹⁷ |

**TABLE 8**

| No. | Ra | A | Q | No. | Ra | A | Q |
|---|---|---|---|---|---|---|---|
| 477 | Ra⁶ | A⁴ | Q¹ | 511 | Ra⁷ | A¹ | Q¹ |
| 478 | Ra⁶ | A⁴ | Q² | 512 | Ra⁷ | A¹ | Q² |
| 479 | Ra⁶ | A⁴ | Q³ | 513 | Ra⁷ | A¹ | Q³ |
| 480 | Ra⁶ | A⁴ | Q⁴ | 514 | Ra⁷ | A¹ | Q⁴ |
| 481 | Ra⁶ | A⁴ | Q⁵ | 515 | Ra⁷ | A¹ | Q⁵ |
| 482 | Ra⁶ | A⁴ | Q⁶ | 516 | Ra⁷ | A¹ | Q⁶ |
| 483 | Ra⁶ | A⁴ | Q⁷ | 517 | Ra⁷ | A¹ | Q⁷ |
| 484 | Ra⁶ | A⁴ | Q⁸ | 518 | Ra⁷ | A¹ | Q⁸ |
| 485 | Ra⁶ | A⁴ | Q⁹ | 519 | Ra⁷ | A¹ | Q⁹ |
| 486 | Ra⁶ | A⁴ | Q¹⁰ | 520 | Ra⁷ | A¹ | Q¹⁰ |
| 487 | Ra⁶ | A⁴ | Q¹¹ | 521 | Ra⁷ | A¹ | Q¹¹ |
| 488 | Ra⁶ | A⁴ | Q¹² | 522 | Ra⁷ | A¹ | Q¹² |
| 489 | Ra⁶ | A⁴ | Q¹³ | 523 | Ra⁷ | A¹ | Q¹³ |
| 490 | Ra⁶ | A⁴ | Q¹⁴ | 524 | Ra⁷ | A¹ | Q¹⁴ |
| 491 | Ra⁶ | A⁴ | Q¹⁵ | 525 | Ra⁷ | A¹ | Q¹⁵ |
| 492 | Ra⁶ | A⁴ | Q¹⁶ | 526 | Ra⁷ | A¹ | Q¹⁶ |
| 493 | Ra⁶ | A⁴ | Q¹⁷ | 527 | Ra⁷ | A¹ | Q¹⁷ |
| 494 | Ra⁶ | A⁵ | Q¹ | 528 | Ra⁷ | A² | Q¹ |
| 495 | Ra⁶ | A⁵ | Q² | 529 | Ra⁷ | A² | Q² |
| 496 | Ra⁶ | A⁵ | Q³ | 530 | Ra⁷ | A² | Q³ |
| 497 | Ra⁶ | A⁵ | Q⁴ | 531 | Ra⁷ | A² | Q⁴ |
| 498 | Ra⁶ | A⁵ | Q⁵ | 532 | Ra⁷ | A² | Q⁵ |
| 499 | Ra⁶ | A⁵ | Q⁶ | 533 | Ra⁷ | A² | Q⁶ |
| 500 | Ra⁶ | A⁵ | Q⁷ | 534 | Ra⁷ | A² | Q⁷ |
| 501 | Ra⁶ | A⁵ | Q⁸ | 535 | Ra⁷ | A² | Q⁸ |
| 502 | Ra⁶ | A⁵ | Q⁹ | 536 | Ra⁷ | A² | Q⁹ |
| 503 | Ra⁶ | A⁵ | Q¹⁰ | 537 | Ra⁷ | A² | Q¹⁰ |
| 504 | Ra⁶ | A⁵ | Q¹¹ | 538 | Ra⁷ | A² | Q¹¹ |
| 505 | Ra⁶ | A⁵ | Q¹² | 539 | Ra⁷ | A² | Q¹² |
| 506 | Ra⁶ | A⁵ | Q¹³ | 540 | Ra⁷ | A² | Q¹³ |
| 507 | Ra⁶ | A⁵ | Q¹⁴ | 541 | Ra⁷ | A² | Q¹⁴ |
| 508 | Ra⁶ | A⁵ | Q¹⁵ | 542 | Ra⁷ | A² | Q¹⁵ |
| 509 | Ra⁶ | A⁵ | Q¹⁶ | 543 | Ra⁷ | A² | Q¹⁶ |
| 510 | Ra⁶ | A⁵ | Q¹⁷ | 544 | Ra⁷ | A² | Q¹⁷ |

**TABLE 9**

| No. | Ra | A | Q | No. | Ra | A | Q |
|---|---|---|---|---|---|---|---|
| 545 | Ra⁷ | A³ | Q¹ | 579 | Ra⁷ | A⁵ | Q¹ |
| 546 | Ra⁷ | A³ | Q² | 580 | Ra⁷ | A⁵ | Q² |
| 547 | Ra⁷ | A³ | Q³ | 581 | Ra⁷ | A⁵ | Q³ |
| 548 | Ra⁷ | A³ | Q⁴ | 582 | Ra⁷ | A⁵ | Q⁴ |
| 549 | Ra⁷ | A³ | Q⁵ | 583 | Ra⁷ | A⁵ | Q⁵ |
| 550 | Ra⁷ | A³ | Q⁶ | 584 | Ra⁷ | A⁵ | Q⁶ |
| 551 | Ra⁷ | A³ | Q⁷ | 585 | Ra⁷ | A⁵ | Q⁷ |
| 552 | Ra⁷ | A³ | Q⁸ | 586 | Ra⁷ | A⁵ | Q⁸ |
| 553 | Ra⁷ | A³ | Q⁹ | 587 | Ra⁷ | A⁵ | Q⁹ |
| 554 | Ra⁷ | A³ | Q¹⁰ | 588 | Ra⁷ | A⁵ | Q¹⁰ |
| 555 | Ra⁷ | A³ | Q¹¹ | 589 | Ra⁷ | A⁵ | Q¹¹ |
| 556 | Ra⁷ | A³ | Q¹² | 590 | Ra⁷ | A⁵ | Q¹² |
| 557 | Ra⁷ | A³ | Q¹³ | 591 | Ra⁷ | A⁵ | Q¹³ |
| 558 | Ra⁷ | A³ | Q¹⁴ | 592 | Ra⁷ | A⁵ | Q¹⁴ |
| 559 | Ra⁷ | A³ | Q¹⁵ | 593 | Ra⁷ | A⁵ | Q¹⁵ |
| 560 | Ra⁷ | A³ | Q¹⁶ | 594 | Ra⁷ | A⁵ | Q¹⁶ |
| 561 | Ra⁷ | A³ | Q¹⁷ | 595 | Ra⁷ | A⁵ | Q¹⁷ |
| 562 | Ra⁷ | A⁴ | Q¹ | 596 | Ra⁸ | A¹ | Q¹ |
| 563 | Ra⁷ | A⁴ | Q² | 597 | Ra⁸ | A¹ | Q² |
| 564 | Ra⁷ | A⁴ | Q³ | 598 | Ra⁸ | A¹ | Q³ |
| 565 | Ra⁷ | A⁴ | Q⁴ | 599 | Ra⁸ | A¹ | Q⁴ |
| 566 | Ra⁷ | A⁴ | Q⁵ | 600 | Ra⁸ | A¹ | Q⁵ |
| 567 | Ra⁷ | A⁴ | Q⁶ | 601 | Ra⁸ | A¹ | Q⁶ |
| 568 | Ra⁷ | A⁴ | Q⁷ | 602 | Ra⁸ | A¹ | Q⁷ |
| 569 | Ra⁷ | A⁴ | Q⁸ | 603 | Ra⁸ | A¹ | Q⁸ |
| 570 | Ra⁷ | A⁴ | Q⁹ | 604 | Ra⁸ | A¹ | Q⁹ |
| 571 | Ra⁷ | A⁴ | Q¹⁰ | 605 | Ra⁸ | A¹ | Q¹⁰ |
| 572 | Ra⁷ | A⁴ | Q¹¹ | 606 | Ra⁸ | A¹ | Q¹¹ |
| 573 | Ra⁷ | A⁴ | Q¹² | 607 | Ra⁸ | A¹ | Q¹² |
| 574 | Ra⁷ | A⁴ | Q¹³ | 608 | Ra⁸ | A¹ | Q¹³ |
| 575 | Ra⁷ | A⁴ | Q¹⁴ | 609 | Ra⁸ | A¹ | Q¹⁴ |
| 576 | Ra⁷ | A⁴ | Q¹⁵ | 610 | Ra⁸ | A¹ | Q¹⁵ |
| 577 | Ra⁷ | A⁴ | Q¹⁶ | 611 | Ra⁸ | A¹ | Q¹⁶ |
| 578 | Ra⁷ | A⁴ | Q¹⁷ | 612 | Ra⁸ | A¹ | Q¹⁷ |

**TABLE 10**

| No. 1 | Ra | A | Q | No. | Ra | A | Q |
|---|---|---|---|---|---|---|---|
| 613 | Ra⁸ | A² | Q¹ | 647 | Ra⁸ | A⁴ | Q¹ |
| 614 | Ra⁸ | A² | Q² | 648 | Ra⁸ | A⁴ | Q² |
| 615 | Ra⁸ | A² | Q³ | 649 | Ra⁸ | A⁴ | Q³ |
| 616 | Ra⁸ | A² | Q⁴ | 650 | Ra⁸ | A⁴ | Q⁴ |
| 617 | Ra⁸ | A² | Q⁵ | 651 | Ra⁸ | A⁴ | Q⁵ |
| 618 | Ra⁸ | A² | Q⁶ | 652 | Ra⁸ | A⁴ | Q⁶ |
| 619 | Ra⁸ | A² | Q⁷ | 653 | Ra⁸ | A⁴ | Q⁷ |
| 620 | Ra⁸ | A² | Q⁸ | 654 | Ra⁸ | A⁴ | Q⁸ |
| 621 | Ra⁸ | A² | Q⁹ | 655 | Ra⁸ | A⁴ | Q⁹ |
| 622 | Ra⁸ | A² | Q¹⁰ | 656 | Ra⁸ | A⁴ | Q¹⁰ |
| 623 | Ra⁸ | A² | Q¹¹ | 657 | Ra⁸ | A⁴ | Q¹¹ |
| 624 | Ra⁸ | A² | Q¹² | 658 | Ra⁸ | A⁴ | Q¹² |
| 625 | Ra⁸ | A² | Q¹³ | 659 | Ra⁸ | A⁴ | Q¹³ |
| 626 | Ra⁸ | A² | Q¹⁴ | 660 | Ra⁸ | A⁴ | Q¹⁴ |
| 627 | Ra⁸ | A² | Q¹⁵ | 661 | Ra⁸ | A⁴ | Q¹⁵ |
| 628 | Ra⁸ | A² | Q¹⁶ | 662 | Ra⁸ | A⁴ | Q¹⁶ |
| 629 | Ra⁸ | A² | Q¹⁷ | 663 | Ra⁸ | A⁴ | Q¹⁷ |
| 630 | Ra⁸ | A³ | Q¹ | 664 | Ra⁸ | A⁵ | Q¹ |
| 631 | Ra⁸ | A³ | Q² | 665 | Ra⁸ | A⁵ | Q² |
| 632 | Ra⁸ | A³ | Q³ | 666 | Ra⁸ | A⁵ | Q³ |
| 633 | Ra⁸ | A³ | Q⁴ | 667 | Ra⁸ | A⁵ | Q⁴ |
| 634 | Ra⁸ | A³ | Q⁵ | 668 | Ra⁸ | A⁵ | Q⁵ |
| 635 | Ra⁸ | A³ | Q⁶ | 669 | Ra⁸ | A⁵ | Q⁶ |
| 636 | Ra⁸ | A³ | Q⁷ | 670 | Ra⁸ | A⁵ | Q⁷ |
| 637 | Ra⁸ | A³ | Q⁸ | 671 | Ra⁸ | A⁵ | Q⁸ |
| 638 | Ra⁸ | A³ | Q⁹ | 672 | Ra⁸ | A⁵ | Q⁹ |
| 639 | Ra⁸ | A³ | Q¹⁰ | 673 | Ra⁸ | A⁵ | Q¹⁰ |
| 640 | Ra⁸ | A³ | Q¹¹ | 674 | Ra⁸ | A⁵ | Q¹¹ |
| 641 | Ra⁸ | A³ | Q¹² | 675 | Ra⁸ | A⁵ | Q¹² |
| 642 | Ra⁸ | A³ | Q¹³ | 676 | Ra⁸ | A⁵ | Q¹³ |
| 643 | Ra⁸ | A³ | Q¹⁴ | 677 | Ra⁸ | A⁵ | Q¹⁴ |
| 644 | Ra⁸ | A³ | Q¹⁵ | 678 | Ra⁸ | A⁵ | Q¹⁵ |
| 645 | Ra⁸ | A³ | Q¹⁶ | 679 | Ra⁸ | A⁵ | Q¹⁶ |
| 646 | Ra⁸ | A³ | Q¹⁷ | 680 | Ra⁸ | A⁵ | Q¹⁷ |

**TABLE 11**

| No. | Ra | A | Q | No. | Ra | A | Q |
|---|---|---|---|---|---|---|---|
| 681 | Ra⁹ | A¹ | Q¹ | 715 | Ra⁹ | A³ | Q¹ |
| 682 | Ra⁹ | A¹ | Q² | 716 | Ra⁹ | A³ | Q² |
| 683 | Ra⁹ | A¹ | Q³ | 717 | Ra⁹ | A³ | Q³ |
| 684 | Ra⁹ | A¹ | Q⁴ | 718 | Ra⁹ | A³ | Q⁴ |
| 685 | Ra⁹ | A¹ | Q⁵ | 719 | Ra⁹ | A³ | Q⁵ |
| 686 | Ra⁹ | A¹ | Q⁶ | 720 | Ra⁹ | A³ | Q⁶ |
| 687 | Ra⁹ | A¹ | Q⁷ | 721 | Ra⁹ | A³ | Q⁷ |
| 688 | Ra⁹ | A¹ | Q⁸ | 722 | Ra⁹ | A³ | Q⁸ |
| 689 | Ra⁹ | A¹ | Q⁹ | 723 | Ra⁹ | A³ | Q⁹ |
| 690 | Ra⁹ | A¹ | Q¹⁰ | 724 | Ra⁹ | A³ | Q¹⁰ |
| 691 | Ra⁹ | A¹ | Q¹¹ | 725 | Ra⁹ | A³ | Q¹¹ |
| 692 | Ra⁹ | A¹ | Q¹² | 726 | Ra⁹ | A³ | Q¹² |
| 693 | Ra⁹ | A¹ | Q¹³ | 727 | Ra⁹ | A³ | Q¹³ |
| 694 | Ra⁹ | A¹ | Q¹⁴ | 728 | Ra⁹ | A³ | Q¹⁴ |
| 695 | Ra⁹ | A¹ | Q¹⁵ | 729 | Ra⁹ | A³ | Q¹⁵ |
| 696 | Ra⁹ | A¹ | Q¹⁶ | 730 | Ra⁹ | A³ | Q¹⁶ |
| 697 | Ra⁹ | A¹ | Q¹⁷ | 731 | Ra⁹ | A³ | Q¹⁷ |
| 698 | Ra⁹ | A² | Q¹ | 732 | Ra⁹ | A⁴ | Q¹ |
| 699 | Ra⁹ | A² | Q² | 733 | Ra⁹ | A⁴ | Q² |
| 700 | Ra⁹ | A² | Q³ | 734 | Ra⁹ | A⁴ | Q³ |
| 701 | Ra⁹ | A² | Q⁴ | 735 | Ra⁹ | A⁴ | Q⁴ |
| 702 | Ra⁹ | A² | Q⁵ | 736 | Ra⁹ | A⁴ | Q⁵ |
| 703 | Ra⁹ | A² | Q⁶ | 737 | Ra⁹ | A⁴ | Q⁶ |
| 704 | Ra⁹ | A² | Q⁷ | 738 | Ra⁹ | A⁴ | Q⁷ |
| 705 | Ra⁹ | A² | Q⁸ | 739 | Ra⁹ | A⁴ | Q⁸ |
| 706 | Ra⁹ | A² | Q⁹ | 740 | Ra⁹ | A⁴ | Q⁹ |
| 707 | Ra⁹ | A² | Q¹⁰ | 741 | Ra⁹ | A⁴ | Q¹⁰ |
| 708 | Ra⁹ | A² | Q¹¹ | 742 | Ra⁹ | A⁴ | Q¹¹ |
| 709 | Ra⁹ | A² | Q¹² | 743 | Ra⁹ | A⁴ | Q¹² |
| 710 | Ra⁹ | A² | Q¹³ | 744 | Ra⁹ | A⁴ | Q¹³ |
| 711 | Ra⁹ | A² | Q¹⁴ | 745 | Ra⁹ | A⁴ | Q¹⁴ |
| 712 | Ra⁹ | A² | Q¹⁵ | 746 | Ra⁹ | A⁴ | Q¹⁵ |
| 713 | Ra⁹ | A² | Q¹⁶ | 747 | Ra⁹ | A⁴ | Q¹⁶ |
| 714 | Ra⁹ | A² | Q¹⁷ | 748 | Ra⁹ | A⁴ | Q¹⁷ |

**TABLE 12**

| No. | Ra | A | Q | No. | Ra | A | Q |
|---|---|---|---|---|---|---|---|
| 749 | Ra⁹ | A⁵ | Q¹ | 783 | Ra¹⁰ | A² | Q¹ |
| 750 | Ra⁹ | A⁵ | Q² | 784 | Ra¹⁰ | A² | Q² |
| 751 | Ra⁹ | A⁵ | Q³ | 785 | Ra¹⁰ | A² | Q³ |
| 752 | Ra⁹ | A⁵ | Q⁴ | 786 | Ra¹⁰ | A² | Q⁴ |
| 753 | Ra⁹ | A⁵ | Q⁵ | 787 | Ra¹⁰ | A² | Q⁵ |
| 754 | Ra⁹ | A⁵ | Q⁶ | 788 | Ra¹⁰ | A² | Q⁶ |
| 755 | Ra⁹ | A⁵ | Q⁷ | 789 | Ra¹⁰ | A² | Q⁷ |
| 756 | Ra⁹ | A⁵ | Q⁸ | 790 | Ra¹⁰ | A² | Q⁸ |
| 757 | Ra⁹ | A⁵ | Q⁹ | 791 | Ra¹⁰ | A² | Q⁹ |
| 758 | Ra⁹ | A⁵ | Q¹⁰ | 792 | Ra¹⁰ | A² | Q¹⁰ |
| 759 | Ra⁹ | A⁵ | Q¹¹ | 793 | Ra¹⁰ | A² | Q¹¹ |
| 760 | Ra⁹ | A⁵ | Q¹² | 794 | Ra¹⁰ | A² | Q¹² |
| 761 | Ra⁹ | A⁵ | Q¹³ | 795 | Ra¹⁰ | A² | Q¹³ |
| 762 | Ra⁹ | A⁵ | Q¹⁴ | 796 | Ra¹⁰ | A² | Q¹⁴ |
| 763 | Ra⁹ | A⁵ | Q¹⁵ | 797 | Ra¹⁰ | A² | Q¹⁵ |
| 764 | Ra⁹ | A⁵ | Q¹⁶ | 798 | Ra¹⁰ | A² | Q¹⁶ |
| 765 | Ra⁹ | A⁵ | Q¹⁷ | 799 | Ra¹⁰ | A² | Q¹⁷ |
| 766 | Ra¹⁰ | A¹ | Q¹ | 800 | Ra¹⁰ | A³ | Q¹ |
| 767 | Ra¹⁰ | A¹ | Q² | 801 | Ra¹⁰ | A³ | Q² |
| 768 | Ra¹⁰ | A¹ | Q³ | 802 | Ra¹⁰ | A³ | Q³ |
| 769 | Ra¹⁰ | A¹ | Q⁴ | 803 | Ra¹⁰ | A³ | Q⁴ |
| 770 | Ra¹⁰ | A¹ | Q⁵ | 804 | Ra¹⁰ | A³ | Q⁵ |
| 771 | Ra¹⁰ | A¹ | Q⁶ | 805 | Ra¹⁰ | A³ | Q⁶ |
| 772 | Ra¹⁰ | A¹ | Q⁷ | 806 | Ra¹⁰ | A³ | Q⁷ |
| 773 | Ra¹⁰ | A¹ | Q⁸ | 807 | Ra¹⁰ | A³ | Q⁸ |
| 774 | Ra¹⁰ | A¹ | Q⁹ | 808 | Ra¹⁰ | A³ | Q⁹ |
| 775 | Ra¹⁰ | A¹ | Q¹⁰ | 809 | Ra¹⁰ | A³ | Q¹⁰ |
| 776 | Ra¹⁰ | A¹ | Q¹¹ | 810 | Ra¹⁰ | A³ | Q¹¹ |
| 777 | Ra¹⁰ | A¹ | Q¹² | 811 | Ra¹⁰ | A³ | Q¹² |
| 778 | Ra¹⁰ | A¹ | Q¹³ | 812 | Ra¹⁰ | A³ | Q¹³ |
| 779 | Ra¹⁰ | A¹ | Q¹⁴ | 813 | Ra¹⁰ | A³ | Q¹⁴ |
| 780 | Ra¹⁰ | A¹ | Q¹⁵ | 814 | Ra¹⁰ | A³ | Q¹⁵ |
| 781 | Ra¹⁰ | A¹ | Q¹⁶ | 815 | Ra¹⁰ | A³ | Q¹⁶ |
| 782 | Ra¹⁰ | A¹ | Q¹⁷ | 816 | Ra¹⁰ | A³ | Q¹⁷ |

**TABLE 13**

| No. | Ra | A | Q | No. | Ra | A | Q |
|---|---|---|---|---|---|---|---|
| 817 | Ra⁹ | A⁴ | Q¹ | 834 | Ra¹⁰ | A⁵ | Q¹ |
| 818 | Ra⁹ | A⁴ | Q² | 835 | Ra¹⁰ | A⁵ | Q² |
| 819 | Ra⁹ | A⁴ | Q³ | 836 | Ra¹⁰ | A⁵ | Q³ |
| 820 | Ra⁹ | A⁴ | Q⁴ | 837 | Ra¹⁰ | A⁵ | Q⁴ |
| 821 | Ra⁹ | A⁴ | Q⁵ | 838 | Ra¹⁰ | A⁵ | Q⁵ |
| 822 | Ra⁹ | A⁴ | Q⁶ | 839 | Ra¹⁰ | A⁵ | Q⁶ |
| 823 | Ra⁹ | A⁴ | Q⁷ | 840 | Ra¹⁰ | A⁵ | Q⁷ |
| 824 | Ra⁹ | A⁴ | Q⁸ | 841 | Ra¹⁰ | A⁵ | Q⁸ |
| 825 | Ra⁹ | A⁴ | Q⁹ | 842 | Ra¹⁰ | A⁵ | Q⁹ |
| 826 | Ra⁹ | A⁴ | Q¹⁰ | 843 | Ra¹⁰ | A⁵ | Q¹⁰ |
| 827 | Ra⁹ | A⁴ | Q¹¹ | 844 | Ra¹⁰ | A⁵ | Q¹¹ |
| 828 | Ra⁹ | A⁴ | Q¹² | 845 | Ra¹⁰ | A⁵ | Q¹² |
| 829 | Ra⁹ | A⁴ | Q¹³ | 846 | Ra¹⁰ | A⁵ | Q¹³ |
| 830 | Ra⁹ | A⁴ | Q¹⁴ | 847 | Ra¹⁰ | A⁵ | Q¹⁴ |
| 831 | Ra⁹ | A⁴ | Q¹⁵ | 848 | Ra¹⁰ | A⁵ | Q¹⁵ |
| 832 | Ra⁹ | A⁴ | Q¹⁶ | 849 | Ra¹⁰ | A⁵ | Q¹⁶ |
| 833 | Ra⁹ | A⁴ | Q¹⁷ | 850 | Ra¹⁰ | A⁵ | Q¹⁷ |

The compounds of the present invention represented by the formula (I) may be converted to a pharmaceutically acceptable salt or may be liberated from the resulting salt, if necessary. The pharmaceutically acceptable salt of the present invention may be, for example, a salt with an alkali metal (such as lithium, sodium and potassium), an alkaline earth metal (such as magnesium and calcium), ammonium, an organic base or an amino acid. It may be a salt with an inorganic acid (such as hydrochloric acid, hydrobromic acid, phosphoric acid and sulfuric acid) or an organic acid (such as acetic acid, citric acid, maleic acid, fumaric acid, benzenesulfonic acid and p-toluenesulfonic acid). A compound of the present invention represented by the formula (I) or a pharmaceutically acceptable salt thereof may be in the form of arbitrary crystals or an arbitrary hydrate, depending on the production conditions. The present invention covers these crystals, hydrates and mixtures. They may be in the form of a solvate with an organic solvent such as acetone, ethanol and tetrahydrofuran, and the present invention covers any of these forms.

In the present invention, the compounds of the present invention represented by the formula (I) may be present in the form of tautomers or geometrical isomers generated by endocyclic or exocyclic isomerization, mixtures of tautomers or geometric isomers or mixtures of thereof. When the compounds of the present invention has an asymmetric center, whether or not resulting from an isomerization, the compounds of the present invention may be in the form of resolved optical isomers or in the form of mixtures containing them in certain ratios.

The compounds which serve as prodrugs are derivatives of the present invention having chemically or metabolically degradable groups which give pharmacologically active compounds of the present invention upon solvolysis or under physiological conditions in vivo. Methods for selecting or producing appropriate prodrugs are disclosed, for example, in Design of Prodrugs (Elsevier, Amsterdam 1985). In the present invention, when the compound has a hydroxy group, acyloxy derivatives obtained by reacting the compound with appropriate acyl halides or appropriate acid anhydrides may, for example, be mentioned as prodrugs. Acyloxys particularly preferred as prodrugs include -OCOC₂H₅, -OCO(t-Bu), -OCOC₁₅H₃₁, -OCO(m-CO₂Na-Ph), -OCOCH₂CH₂CO₂Na, -OCOCH(NH₂)CH₃, -OCOCH₂N(CH₃)₂ and the like. When the compounds of the present invention have an amino group, amide derivatives obtained by reacting the compound having an amino group with appropriate acid halides or appropriate mixed acid anhydrides may, for example, be mentioned as prodrugs. Amides particularly preferred as prodrugs include -NHCO(CH₂)₂₀OCH₃, -NHCOCH(NH₂)CH₃ and the like.

The specific compound to used in the method of the present invention can be synthesized chemically by reference to Patent Documents WO2004/108683, WO2006/064957, WO2007/010954, WO2010/140685 and the like, though there are no particular restrictions.

### EXAMPLES

Now, the present invention will be described in further detail with reference to Examples. However, it should be understood that the present invention is by no means restricted by these specific Examples.

The CO₂ concentration (%) in the CO₂ incubator is expressed in the percentage of the volume of CO₂ in the atmosphere. PBS denotes phosphate buffered saline (Sigma-Aldrich Japan), and FBS denotes fetal bovine serum.

### (TEST EXAMPLE 1: Preparation of human iPS cell-derived sac-like structures (iPS-sacs))

In this Example, an iPS cell line TkDA3-4 (established by Tokyo University by introducing Oct3/4,Klf4,Sox2 and c-Myc into skin cells: see: WO2009122747) was used. As the feeder cells, a mouse embryo-derived cell line C3H10T1/2, provided by BloResource center, Riken Tsukuba Institute, was used. On the day before the differentiation experiment, C3H10T1/2 cells were irradiated with 50 Gy radiation, seeded on dishes coated with 0.1% gelatin at a density of from 6 to 8×10⁵/10cm dish and used as feeder cells.

Human iPS cells were seeded on the C3H10T1/2 cells and cultured in IMDM (Invitrogen/GIBCO) supplemented with 15% FBS (JRH BIOSCIENCES, U.S.A), 2 mM L-glutamine (Invitrogen), 100 Unit/mL Penicillin-**100µg/mL Streptmycin** (Sigma), ITS supplement (10 **µg/mL** insulin, 5.5mg/mL transferrin, 5 ng/mL sodium selenite) (Sigma), 50 **µg/mL** ascorbic acid (Sigma), 0.45 mM MTG (Sigma) and 20 ng/mL VEGF (R&D systems) and incubated in 5% CO₂ at 37°C.

After about 14 to 15 days of incubation, a number of sac-like structures (iPS-sacs) containing blood cell-like cells were observed.

### (TEST EXAMPLE 2: Induction of megakaryocytes / platelets from the sac-like structures (iPS-sacs))

Next, the sac-like structures were physically disrupted with a 10mL disposable pipette, and hematopoietic progenitor cells and sac-like structures were separated by using a 70 µm cell strainer. The hematopoietic progenitor cells were seeded on irradiated C3H10T1/2 cells (from 6 to 8×10⁵cells/6-well plate) newly prepared on a 6-well plate, at a density of 3×10⁴ cells/well and incubated in IMDM (Invitrogen/GIBCO) supplemented with 15% FBS (JRH BIOSCIENCES, U.S.A), 2 mM L-glutamine (Invitrogen), 100 Unit/mL Penicillin-**100µg/mL Streptmysin** (Sigma), ITS supplement (10 **µg/mL** insulin, 5.5 mg/mL transferrin, 5 ng/mL sodium selenite) (Sigma), 50 **µg/mL** ascorbic acid (Sigma), 0.45 mM monothioglycerol (MTG, Sigma) and 100 ng/mL human TPO (Peprotec) or one of the following specific compounds (100 ng/mL No.1, 30 ng/mL No.2, 50 ng/mL No.3, 1000 ng/mL No.4, 100 ng/mL No.5, 100 ng/mL No.6, 300 ng/mL No.7, 300 ng/mL No.8) to induce megakaryocytes / platelets.

The names and structural formulae of the specific compounds used in this Example are given below.
Specific Compound No.1:(E)-5-(2-{1-[5-(3,4-dichlorophenyl)-4-hydroxythiophen-3-yl]ethyliden}hydrazinecarbonyl)-N-[4-(2-hydroxyethylcarbamoyl)benzyl]thiophene-2-carboxamide
Specific Compound No.2:(E)-5-(2-{1-[5-(4-bromophenyl)-4-hydroxythiophen-3-yl]ethylidene}hydrazinecarbonyl)-N-[4-(2- hydroxyethylcarbamoyl)benzyl]thiophene-2-carboxamide
Specific Compound No.3:(E)-5-(2-{1-[5-(3,4-dichlorophenyl)-4-hydroxythiophen-3-yl]ethylidene}hydrazinecarbonyl)-N-(pyridin-4-ylmethyl)thiophene-2-carboxamide.
Specific Compound No.4 (Comparative):(E)-5-(2-{1-[5-(2,3-dihydro-1H-inden-5-yl)-4-hydroxythiophen-3-yl]ethylidene}hydrazinecarbonyl)thiophene-2-carboxylic acid
Specific Compound No.5: potassium (E)-2-(3,4-dichlorophenyl)-4-[1-(2-{5-[(pyrazin-2-ylmethyl)carbamoy]thiophene-2-carbonyl}hydrazono)ethyl]thiophen-3-olate
Specific Compound No.6:(E)-5-(2-{1-[5-(4-bromophenyl)-4-hydroxythiophen-3-yl]ethylidene}hydrazinecarbonyl)-N-{4-[2-(piperazin-1-yl)ethylcarbamoyl]benzyl}thiophene-2-carboxamide
Specific Compound No.7:(E)-N-[4-(2-amino-2-oxoethyl)benzyl]-5-(2-{1-[5-(3,4-dichlorophenyl)-4-hydroxythiophen-3-yl]ethylidene}hydrazinecarbonyl)thiophene-2-carboxamide
Specific Compound No.8:(E)-N-(4-{2-[bis(2-hydroxyethyl)amino]ethylcarbamoyl}benzyl)-5-(2-{1-[5-(4-t-butylphenyl)-4-hydroxythiophen-3-yl]ethylidene}hydrazinecarbonyl)thiophene-2-carboxamide.

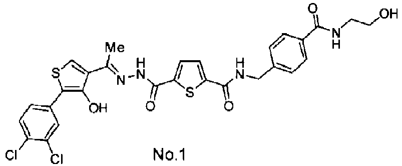

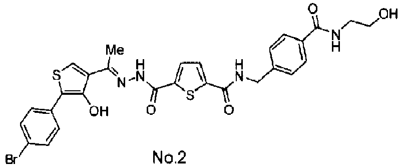

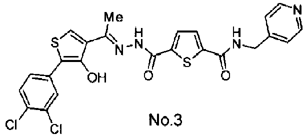

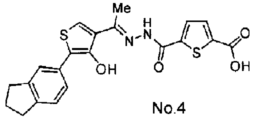

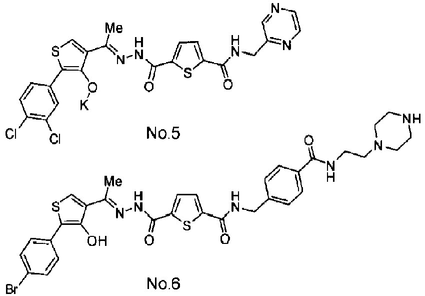

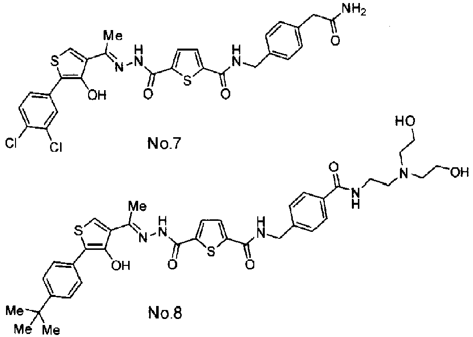

These compounds were synthesized by known procedures in accordance with, WO2007/010954, WO2010/140685, WO2011/049213 and the like.

### (TEST EXAMPLE 3: Megakaryocyte / platelet counts in cell cultures)

The nonadherent cells in the 23- to 24-day TkDA3-4 cultures were characterized by cell surface antigens with a fluocytometer (Becton, Dickinson and Company, BDFACSAia) after addition of 8.5 mM sodium citrate (Sigma), 6.5 mM citric acid (Sigma), 10.4 mM glucose (Sigma), anti-human CD41a antibody (Becton, Dickinson and Company) and anti-human CD42b antibody (BioLegend) in terms of final concentration. Platelets were sorted out by size with a flow cytometer and counted by using BD Trucount tubes (Becton, Dickinson and Company). Megakaryocytes were sorted by size from platelets by centrifugation (310 g, 5 minutes) and flow cytometry and counted with a hemocytometer. The megakaryocytes and platelets were positive for the cell surface antigens specific to megakaryocytes and platelets, human CD41a (integrin **αIIb**) and human CD42b (**GPIbα**) (Figs. 1 and 2; megakaryocytes, Figs. 3 and 4; platelets). The specific compounds of the present invention showed higher megakaryopoietic and thrombopoietic effects than TPO.

### (TEST EXAMPLE 4: Functional analysis of the platelets)

Next, activation of integrin by platelet activators was examined. From nonadherent cells in 23- or 24-day culture of TkDA3-4 cells, nucleate cells were removed, and platelets were separated by centrifugation (400 g, 10 minutes) and treated with human anti-CD42b antibody (BioLegend), FITC-labeled PAC-1 (Becton, Dickinson and Company) and 500 **µM** a platelet activator, adenosine diphosphate (ADP, Sigma). 15 minutes later, the binding of PAC-1 as a platelet activation maker to palatelets was analyzed with a flow cytometer and expressed as the mean fluorescence intensity (MFI). As a result, the platelets derived from human iPS cells by using the specific compounds of the present invention showed as much activation of the integrin (binding of PAC-1 to platelets) as platelets from peripheral blood. The results demonstrate that platelets derived from iPS cells by using a specific compound of the present invention are as functional as platelets from peripheral blood.

### (TEST EXAMPLE 5 (Comparative): Preparation of human ES cell-derived sac-like structures (ES-sacs))

In this Example, an ES cell line KhES-3 (established by Kyoto University, see: Biochem Biophys Res Commun. 2006. 345: 926-932) was used. As the feeder cells, a mouse embryo-derived cell line C3H10T1/2, provided by BloResource center, Riken Tsukuba Institute, was used. On the day before the differentiation experiment, C3H10T1/2 cells were irradiated with 50 Gy radiation, seeded on dishes coated with 0.1% gelatin at a density of from 6 to 8×10⁵/10 cm dish and used as feeder cells.

Human ES cells were seeded on the C3H10T1/2 cells and cultured in IMDM (Invitrogen/GIBCO) supplemented with 15% FBS (JRH BIOSCIENCES, U.S.A), 2 mM L-glutamine (Invitrogen), 100 Unit/mL Penicillin-100 **µg/mL Streptmycin** (Sigma), ITS supplement (10 **µg/mL** insulin, 5.5 mg/mL transferrin, 5 ng/mL sodium selenite) (Sigma), 50 **µg/mL** ascorbic acid (Sigma), 0.45 mM MTG (Sigma) and 20 ng/mL VEGF (R&D systems) and incubated in 5% CO₂ at 37°C.

After about 14 to 15 days of incubation, a number of sac-like structures (ES-sacs) containing blood cell-like cells were observed.

### (TEST EXAMPLE 6 (Comparative): Induction of megakaryocytes / platelets from the sac-like structures (ES-sacs))

Next, the sac-like structures were mechanically disrupted with a 10 mL disposable pipette, and hematopoietic progenitor cells and sac-like structures were separated by using a 70 µm cell strainer. The hematopoietic progenitor cells were seeded on irradiated C3H10T1/2 cells (from 6 to 8×10⁵ cells/6-well plate) newly prepared on a 6-well plate, at a density of 3×10⁴ cells/well and incubated in IMDM (Invitrogen/GIBCO) supplemented with 15% FBS (JRH BIOSCIENCES, U.S.A), 2 mM L-glutamine (Invitrogen), 100 Unit/mL Penicillin-100 **µg/mL Streptmysin** (Sigma), ITS supplement (10 **µg/mL** insulin, 5.5 mg/mL transferrin, 5 ng/mL sodium selenite) (Sigma), 50 **µg/mL** ascorbic acid (Sigma), 0.45 mM monothioglycerol (MTG, Sigma) and 100 ng/mL human TPO (Peprotec) or a specific compound used in Test Example 2 (30 ng/mL No.2, 50 ng/mL No.3) to induce megakaryocytes / platelets.

### (TEST EXAMPLE 7: Megakaryocyte / platelet counts in cell cultures)

The nonadherent cells in the 23- to 24-day KhES-3 cultures were characterized by cell surface antigens after addition of 8.5 mM sodium citrate (Sigma), 6.5 mM citric acid (Sigma), 10.4 mM glucose (Sigma), human anti-CD41 a antibody (Becton, Dickinson and Company) and human anti-CD42b antibody (BioLegend) in terms of final concentration. Platelets were sorted out by size with a flow cytometer and counted by using BD Trucount tubes (Becton, Dickinson and Company). Megakaryocytes were sorted by size from platelets by centrifugation (310 g, 5 minutes) and flow cytometry and counted with a hemocytometer. The megakaryocytes and platelets were positive for the cell surface antigens specific to megakaryocytes and platelets, human CD41 a (integrin **αIIb**) and human CD42b (**GPIbα**) (Fig. 6; platelet counts). The specific compound used according to the present invention showed higher megakaryopoietic and thrombopoietic effects than TPO did.

### (TEST EXAMPLE 8: Preparation of genetically manipulated hematopoietic progenitor cells)

Hematopoietic progenitor cells were obtained from KhES-3 cell-derived sac-like structures obtained in Test Examples 5 and 6, and cells (Myc-Bmi cell line) showing enhanced proliferative capability in the presence of estradiol through enhanced expression of the oncogene c-Myc and the polycomb gene Bmi1 by using a pMX tet off vector system for regulated gene expression were obtained by using the method described in WO 2011/034073. In the absence of estradiol in the presence of doxycylcline, Myc-Bmi cells show repressed expression of c-Myc and Bmi1 and produce functional platelets, but hardly proliferate. From the Myc-Bmi cells, cells (Myc-Bmi-BCLXL cell line) which show enhanced expression of the apoptosis suppressor gene BCLXL in the absence of estradiol in the presence of doxycycline and can proliferate even in the absence of estradiol in the presence doxycycline by using an Ai-Lv tet on g vector system for regulated gene expression (Clontech) were obtained. Further, expression of p53 gene was suppressed by short hairpin (sh) RNA interference to promote polyploidization in the course of differentiation into mature megakaryocytes. Myc-Bmi-BCLXL cells were transfected with a FG12 lenti virus carrying shp53 to obtain Myc-Bmi-BCLXL cells showing repressed p53 expression (p53KD-Myc-Bmi-BCLXL cell line). The p53KD-Myc-Bmi-BCLXL cells were maintained by culturing on C3H10T1/2 cells inactivated by preliminary treatment with 10 **µg/mL** mitocycin C (Wako Pure Chemical Industries) for 0.5 to 5 hours in IMDM (Invitrogen/GIBCO) supplemented with 15% FBS (Invitrogen/GIBCO), 2m M L-glutamine- 100 Unit/mL Penicillin-100 **µg/mL** Streptmysin (Invitrogen/GIBCO), ITS supplement (10 **µg/mL** insulin, 5.5 mg/mL transferrin, 5 ng/mL sodium selenite) (Invitrogen/GIBCO), 50 **µg/mL** ascorbic acid (Sigma), 0.45 mM monothiglycerol (MTG, Sigma), 10 **µg/mL** doxycycline (Clontech), 50 ng/mL SCF (R&D system) and 100 ng/mL human TPO (R&D system) in 5% CO₂ at 39°C.

### (TEST EXAMPLE 9: Induction of megakaryocytes / platelets from genetically manipulated hematopoietic progenitor cells)

p53KD-Myc-Bmi-BCLXL cells were seeded on C3H10T1/2 cells inactivated by preliminary treatment with 10 **µg/mL** mitocycin C (Wako Pure Chemical Industries) for 0.5 to 5 hours (6∼8×10⁵ cells/6-well plate) and cultured in IMDM (Invitrogen/GIBCO) supplemented with 15% FBS (Invitrogen/GIBCO), 2 mM L-glutamine-100 Unit/mL Penicillin-100 **µg/mL Streptmysin** (Invitrogen/GIBCO), ITS suplement (10 **µg/mL** insulin, 5.5 mg/mL transferin, 5 ng/mL sodium selenite) (Invitrogen/GIBCO), 50 **µg/mL** ascorbic acid (Sigma), 0.45 mM monothioglycerol (MTG, Sigma), 10 **µg/mL** doxycyclin (Clontech), 0.5 mM valproic acid (Sigma), 10 **µM Y--27632** (Wako Pure Chemical Industries), 5 **µM** (S)(-/-)-Blebbistatin (Toronto Research Chemicals), 50 ng/mL SCF (R&D system) and 100 ng/mL human TPO (R&D system) or a specific compound mentioned in Test Example 2 (100 ng/mL No.2 or 50 ng/mL No.3) in 5% CO₂ at 39°C for 7 days to induce mature megakaryocytes and platelets. The nonadherent cells in the 7-day cultures were characterized by cell surface antigens with a flow cytometer (Becton, Dickinson and Company, BDFACSAria) after addition of 8.5 mM sodium citrate (Sigma), 6.5 mM citric acid (Sigma), 10.4 mM glucose (Sigma), anti-human CD41a antigen (Becton, Dickinson and Company), anti-human CD42b antibody (BioLegend) in terms of final concentration. Platelets were sorted out by size with a flow cytometer and counted by using BD Trucount tubes (Becton, Dickinson and Company). The platelets were positive for the cell surface antigens specific to platelets, human CD41a (integrin **αIIb**) and human CD42b (**GPIbα**) (Fig.7; platelets). The specific compounds used according to the present invention showed higher thrombopoietic effect than TPO did.

These results demonstrate that megakaryocytes and platelets are induced efficiently from human iPS cells in accordance with the method of the present invention by using the specific compounds.

### INDUSTRIAL APPLICABILITY

Megakaryocytes and platelets can be expanded from human pluripotent stem cells more efficiently in the presence of a specific compound used according to the present invention as an active ingredient in culture than in its absence or in the presence of TPO. Platelets produced by using a specific compound are useful for diseases accompanied by a decrease in platelets such as hematopoietic dysfunction and tumors, and hence their application to transfusion therapy is expected. According to the present invention, it is possible to provide platelets which can overcome the problem of HLA compatibility. Therefore, it is possible to supply platelets to patients who require transfusion and solve the problem of platelet destruction by generation of anti-platelet antibodies.

## Claims

1. A method for producing megakaryocytes and/or platelets, comprising
(i) forming hematopoietic progenitor cells *ex vivo* in a sac-like structure by differentiating human induced pluripotent stem (iPS) cells, wherein the sac-like structure is a three-dimensional saccular structure containing hematopoietic progenitor cells inside; and
(ii) culturing the hematopoietic progenitor cells separated from the septal cells of the sac-like structure ex *vivo* in the presence of a compound represented by the formula (I), a tautomer, prodrug or pharmaceutically acceptable salt of the compound or a solvate thereof, for differentiating the hematopoietic progenitor cells into megakaryocytes and/or platelets: 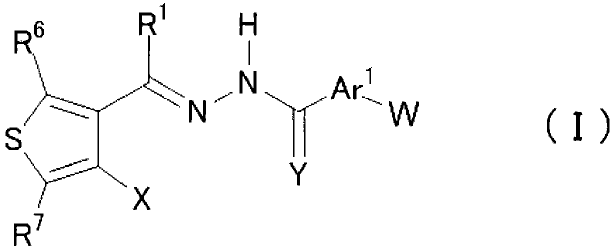 wherein W is a substituent represented by the formula (Ia): 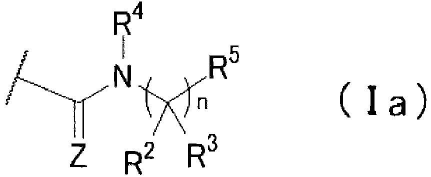
R¹ is a methyl group,
each of R², R³, R⁴ and R⁶ is a hydrogen atom,
n is an integer of 1 or 2,
R⁵ is a phenyl group, a 2-pyridyl group, a 3-pyridyl group, a 4-pyridyl group, a 3-pyridazinyl group, a 4-pyridazinyl group, a 2-pyrimidinyl group, a 4-pyrimidinyl group, a 5-pyrimidinyl group, or a 2-pyrazinyl group which may be substituted with one or more substituents independently represented by V¹, provided that when n is 2, R⁵ is not an unsubstituted pyridyl group,
R⁷ is a phenyl group which may be substituted with one or more substituents selected from methyl groups, t-butyl groups, halogen atoms, methoxy groups, trifluoromethyl groups and trifluoromethoxy groups, or is a substituent represented by any one of the formulae (A01) to (A15): 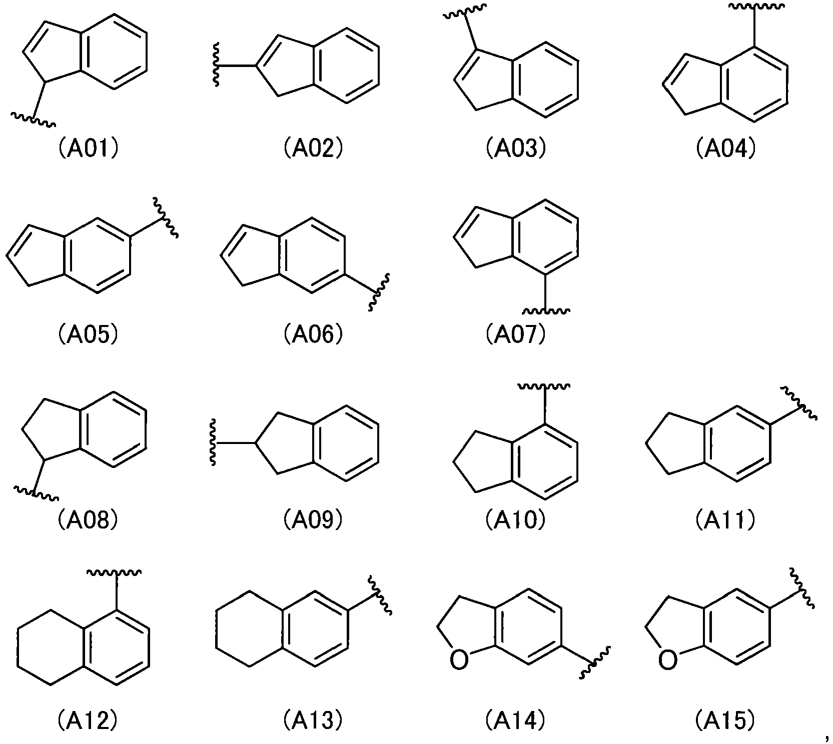
Ar¹ is represented by the formula (IV): 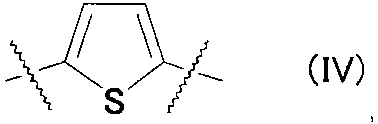
X is -OH,
each of Y and Z is independently an oxygen atom or a sulfur atom, and
V¹ is represented by any one of the formulae (V) to (XXII):

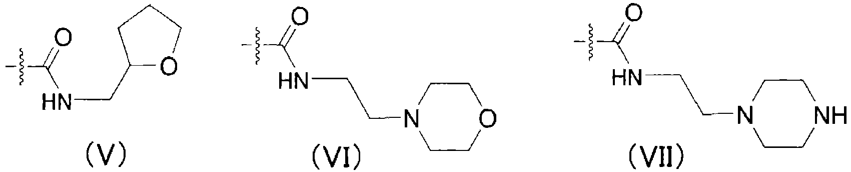

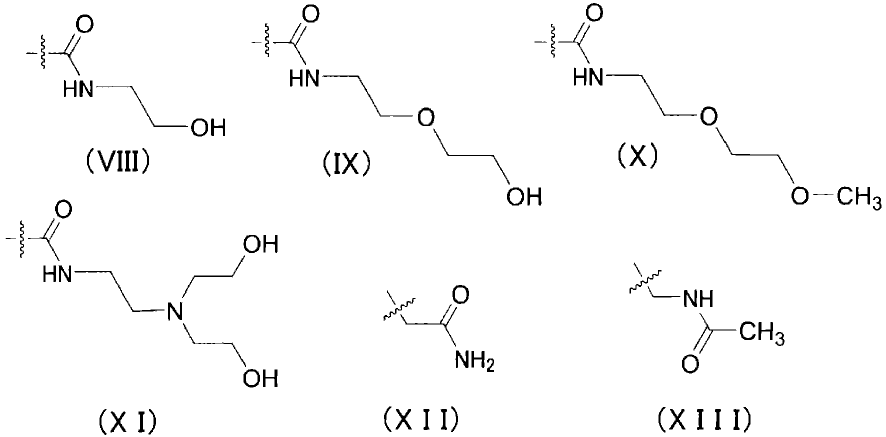

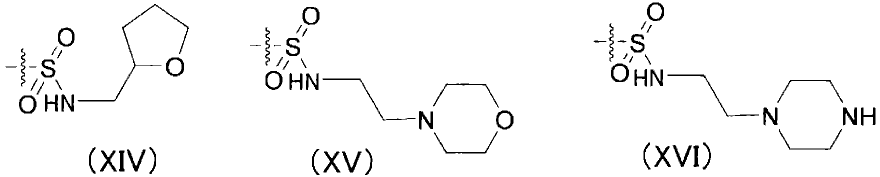

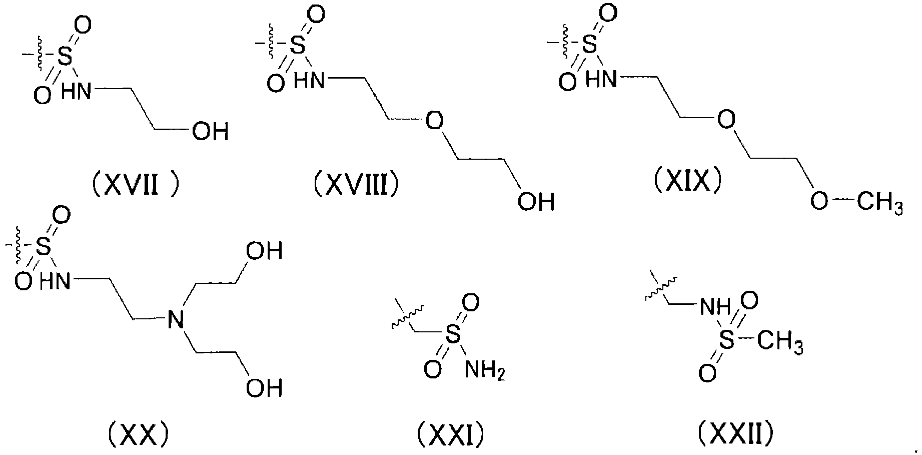

2. The method according to Claim 1, wherein Y and Z are oxygen atoms.

3. The method according to Claim 1, wherein R⁵ is a phenyl group which may be substituted with one or more substituents independently represented by V¹.

4. The method according to Claim 1, wherein R⁵ is a 4-pyridyl group.

5. The method according to Claim 1, wherein R⁵ is a phenyl group substituted with a substituent represented by the formula (VIII):

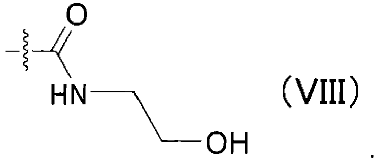

6. The method according to any one of Claims 1 to 5, wherein n is an integer of 1.

7. The method according to any one of Claims 1 to 6, wherein R⁷ is a phenyl group substituted with one or more substituents selected from methyl groups, t-butyl groups, halogen atoms, methoxy groups, trifluoromethyl groups and trifluoromethoxy groups.

8. The method according to any one of Claims 1 to 6, wherein R⁷ is a phenyl group which may be substituted with one or two halogen atoms.

9. The method according to Claim 1, wherein the compound represented by the formula (I) is (E)-5-(2-{1-[5-(3,4-dichlorophenyl)-4-hydroxythiophen-3-yl]ethylidene}hydrazinecarbonyl)-N-[4-(2-hydroxyethylcarbamoyl)benzyl]thiophene-2-carboxamide, (E)-5-(2-{1-[5-(4-bromophenyl)-4-hydroxythiophen-3-yl]ethylidene}hydrazinecarbonyl)-N-[4-(2-hydroxyethylcarbamoyl)benzyl]thiophene-2-carboxamide or (E)-5-(2-{1-[5-(3,4-dichlorophenyl)-4-hydroxylthiophen-3-yl]ethylidene}hydrazinecarbonyl)-N-(pyridin-4-ylmethyl)thiophene-2-carboxamide.

10. The method according to Claim 1, wherein n is an integer of 1,
R⁵ is a pyridyl group, a pyrazinyl group or a phenyl group substituted with a substituent represented by the formula (VII), (VIII), (XI) or (XII): 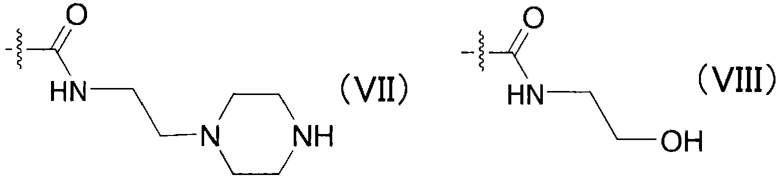 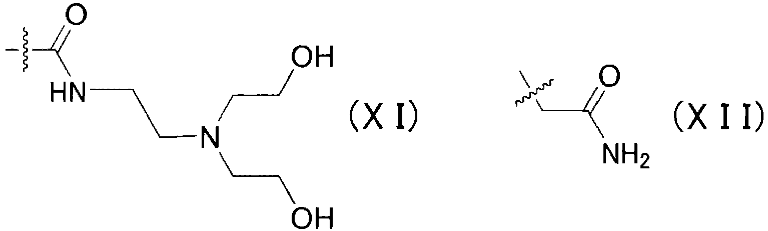 R⁷ is a phenyl group which may be substituted with one or two halogen atoms or a substituent represented by the formula (A11), (A13) or (A15): 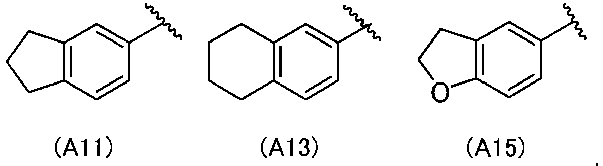 and
Y and Z are oxygen atoms.

11. The method according to Claim 1, wherein the compound represented by the formula (I) is (E)-5-(2-{1-[5-(3,4-dichlorophenyl)-4-hydroxythiophen-3-yl]ethylidene}hydrazinecarbonyl)-N-[4-(2-hydroxyethylcarbamoyl)benzyl]thiophene-2-carboxamide, (E)-5-(2-{1-[5-(4-bromophenyl)-4-hydroxythiophen-3-yl]ethylidene}hydrazinecarbonyl)-N-[4-(2-hydroxyethylcarbamoyl)benzyl]thiophene-2-carboxamide, (E)-5-(2-{1-[5-(3,4-dichlorophenyl)-4-hydroxythiophen-3-yl]ethylidene}hydrazinecarbonyl)-N-(pyridin-4-ylmethyl)thiophene-2-carboxamide, potassium (E)-2-(3,4-dichlorophenyl)-4-[1-(2-{5-[(pyrazin-2-ylmethyl)carbamoyl]thiophene-2-carbonyl}hydrazono)ethyl]thiophen-3-olate, (E)-5-(2-{1-[5-(4-bromophenyl)-4-hydroxythiophen-3-yl]ethylidene}hydrazinecarbonyl)-N-{4-[2-(piperazin-1-yl)ethylcarbamoyl]benzyl}thiophene-2-carboxamide, (E)-N-[4-(2-amino-2-oxoethyl)benzyl]-5-(2-{1-[5-(3,4-dichlorophenyl)-4-hydroxythiophen-3-yl]ethylidene}hydrazinecarbonyl)thiophene-2-carboxamide or (E)-N-(4-{2-[bis(2-hydroxyethyl)amino]ethylcarbamoyl}benzyl)-5-(2-{1-[5-(4-t-butylphenyl)-4-hydroxythiophen-3-yl]ethylidene}hydrazinecarbonyl)thiophene-2-carboxamide.

12. The method according to any one of Claims 1 to 11, wherein the hematopoietic progenitor cells derived from pluripotent stem cells have one or more introduced genes selected from oncogenes, polycomb genes, apoptosis suppressor genes and genes which suppress a tumor suppressor gene and have proliferative and/or differentiative capability enhanced by regulation of expression of the introduced genes.

13. The method according to any one of Claims 1 to 12, wherein the hematopoietic progenitor cells derived from pluripotent stem cells are hematopoietic progenitor cells which have one or more introduced genes selected from MYC family genes, Bmi1 genes, BCL2 family genes and genes which suppress the p53 gene expression and have proliferative and/or differentiative capability enhanced by regulation of expression of the introduced genes.

14. The method according to any one of Claims 1 to 13, wherein the sac-like structures are obtainable by co-culture with feeder cells for an incubation time of 14 to 17 days.

## Patentansprüche

1. Verfahren zur Herstellung von Megakaryozyten und/oder Blutplättchen, umfassend
(i) Bilden von blutbildenden Vorläuferzellen *ex vivo* in einer sackartigen Struktur, durch Differenzieren von humanen induzierten pluripotenten Stammzellen (iSP), wobei die sackartige Struktur eine dreidimensionale sackförmige Struktur ist, die blutbildende Vorläuferzellen im Inneren enthält; und
(ii) Kultivieren der blutbildenden Vorläuferzellen, die von den Nischenzellen der sackartigen Struktur *ex vivo* separiert wurden, in Anwesenheit einer Verbindung der Formel (I), eines Tautomers, eines Pro-Pharmakons oder eines pharmazeutisch verträglichen Salzes der Verbindung oder eines Solvats derselben, um die blutbildenden Vorläuferzellen zu Megakaryozyten und/oder Blutplättchen zu differenzieren: 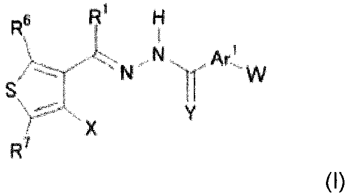 worin W für einen Substituent der Formel (la) steht: 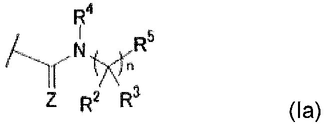
R¹ für eine Methylgruppe steht,
jedes R², R³, R⁴ und R⁶ für ein Wasserstoffatom steht,
n für eine ganze Zahle von 1 oder 2 steht,
R⁵ für eine Phenylgruppe steht, eine 2-Pyridylgruppe, 3-Pyridylgruppe, 4-Pyridylgruppe, eine 3-Pyridazinylgruppe, eine 4-Pyridazinylgruppe, eine 2-Pyrimidinylgruppe, eine 4-Pyrimidinylgruppe, eine 5-Pyrimidinylgruppe oder eine 2-Pyrazinylgruppe, die mit einem oder mehreren Substituenten, die unabhängig durch V¹ wiedergegeben werden, substituiert sein kann, mit der Maßgabe, dass, wenn n für 2 steht, R⁵ keine unsubstituierte Pyridylgruppe ist,
R⁷ für eine Phenylgruppe steht, die mit einem oder mehreren Substituenten, ausgewählt aus Methylgruppen, t-Butylgruppen, Halogenatomen, Methoxygruppen, Trifluormethylgruppen und Trifluormethoxygruppen, substituiert sein kann, oder ein Substituent ist, der durch eine beliebige der Formeln (A01) bis (A15) wiedergegeben 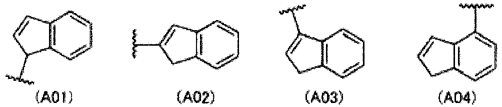 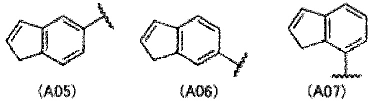 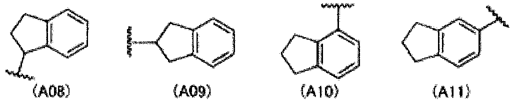 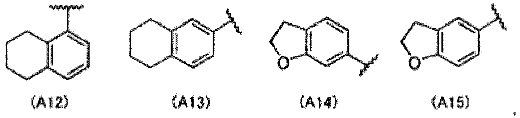 wird:
Ar¹ durch die Formel (IV) wiedergegeben wird: 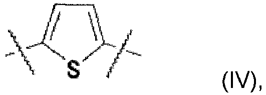
X für OH steht,
jedes Y und Z unabhängig für ein Sauerstoffatom oder ein Schwefelatom steht und
V¹ durch eine beliebige der Formeln (V) bis (XXII) wiedergegeben wird:

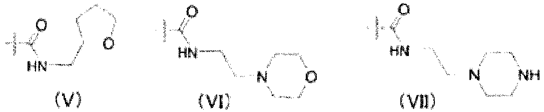

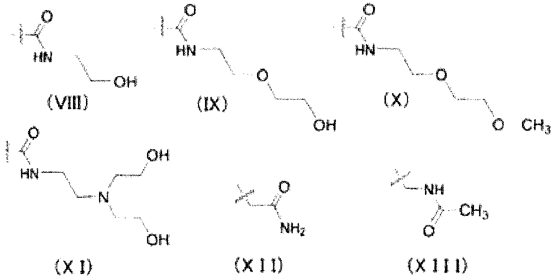

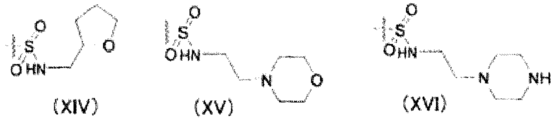

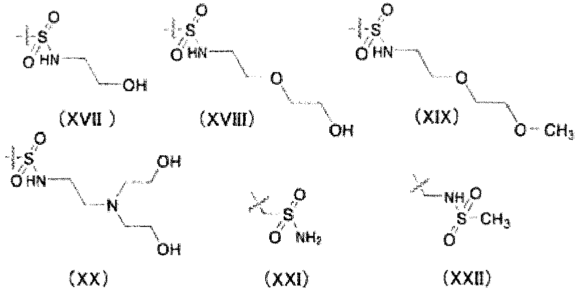

2. Verfahren nach Anspruch 1, wobei Y und Z für Sauerstoffatome stehen.

3. Verfahren nach Anspruch 1, wobei R⁵ für eine Phenylgruppe steht, die mit einem oder mehreren Substituenten substituiert sein kann, die unabhängig durch V¹ wiedergegeben werden.

4. Verfahren nach Anspruch 1, wobei R⁵ für eine 4-Pyridylgruppe steht.

5. Verfahren nach Anspruch 1, wobei R⁵ für eine Phenylgruppe steht, die mit einem Substituent der Formel (VIII) substituiert ist:

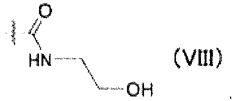

6. Verfahren nach einem der Ansprüche 1 bis 5, wobei n für eine ganze Zahl von 1 steht.

7. Verfahren nach einem der Ansprüche 1 bis 6, wobei R⁷ für eine Phenylgruppe steht, die mit einem oder mehreren Substituenten substituiert ist, ausgewählt aus Methylgruppen, t-Butylgruppen, Halogenatomen, Methoxygruppen, Trifluormethylgruppen und Trifluormethoxygruppen.

8. Verfahren nach einem der Ansprüche 1 bis 6, wobei R⁷ für eine Phenylgruppe steht, die mit einem oder zwei Halogenatomen substituiert sein kann.

9. Verfahren nach Anspruch 1, wobei die Verbindung der Formel (1) (E)-5-(2-{1-[5-(3,4-Dichlorphenyl)-4-hydroxythiophen-3-yl]ethyliden}hydrazincarbonyl)-N-[4-(2-hydroxyethylcarbamoyl)benzyl]thiophen-2-carboxamid, (E)-5-(2-{1-[5-(4-Bromphenyl)-4-hydroxythiophen-3-yl]ethyliden}hydrazincarbonyl)-N-[4-(2-hydroxyethylcarbamoyl)benzyl]thiophen-2-carboxamid oder (E)-5-(2-{1-[5-(3,4-Dichlorphenyl)-4-hydroxylthiophen-3-yl]ethyliden}hydrazincarbonyl)-N-(pyridin-4-ylmethyl)thiophen-2-carboxamid ist.

10. Verfahren nach Anspruch 1, wobei n für eine ganze Zahl von 1 steht, R⁵ für eine Pyridylgruppe, eine Pyrazinylgruppe oder eine Phenylgruppe steht, die mit einem Substituent der Formel (VII), (VIII), (XI) oder (XII) substituiert ist: 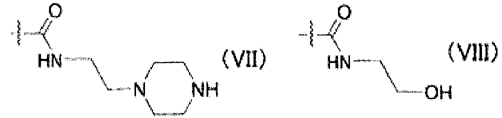 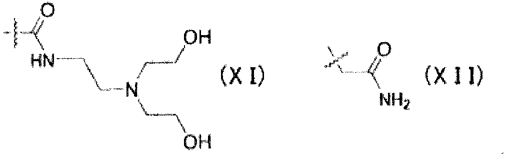 R⁷ für eine Phenylgruppe steht, die mit einem oder zwei Halogenatomen substituiert sein kann oder einem Substituent der Formel (A11), (A13) oder (A15): 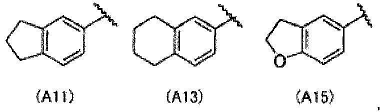 und Y und Z für Sauerstoffatome stehen.

11. Verfahren nach Anspruch 1, wobei die Verbindung der Formel (1) (E)-5-(2-{1-[5-(3,4-Dichlorphenyl)-4-hydroxythiophen-3-yl]ethyliden}hydrazincarbonyl)-N-[4-(2-hydroxyethylcarbamoyl)benzyl]thiophen-2-carboxamid, (E)-5-(2-{1-[5-(4-Bromphenyl)-4-hydroxythiophen-3-yl]ethyliden}hydrazincarbonyl)-N-[4-(2-hydroxyethylcarbamoyl)benzyl]thiophen-2-carboxamid, (E)-5-(2-{1-[5-(3,4-Dichlorphenyl)-4-hydroxythiophen-3-yl]ethyliden}hydrazincarbonyl)-N-(pyridin-4-ylmethyl)thiophen-2-carboxamid, Kalium-(E)-2-(3,4-dichlorphenyl)-4-[1-(2-{5-[(pyrazin-2-ylmethyl)carbamoyl]thiophen-2-carbonyl}hydrazono)ethyl]thiophen-3-olat, (E)-5-(2-{1-[5-(4-Bromphenyl)-4-hydroxythiophen-3-yl]ethyliden}hydrazincarbonyl)-N-{4-[2-(piperazin-1-yl)ethylcarbamoyl]benzyl}thiophen-2-carboxamid, (E)-N-[4-(2-Amino-2-oxoethyl)benzyl]-5-(2-{1-[5-(3,4-dichlorphenyl)-4-hydroxythiophen-3-yl]ethyliden}hydrazincarbonyl)thiophen-2-carboxamid oder (E)-N-(4-{2-[bis(2-Hydroxyethyl)amino]ethylcarbamoyl}benzyl)-5-(2-{1-[5-(4-t-butylphenyl)-4-hydroxythiophen-3-yl]ethyliden}hydrazincarbonyl)thiophen-2-carboxamid ist.

12. Verfahren nach einem der Ansprüche 1 bis 11, wobei die blutbildenden Vorläuferzellen, die von den pluripotenten Stammzellen stammen, ein oder mehrere eingeführte Gene aufweisen, ausgewählt aus Onkogenen, Polycomb-Genen, Apoptose-Suppressor-Genen und Genen, die ein Tumor-Suppressor-Gen unterdrücken, und eine proliferative und/oder differenzierende Fähigkeit aufweisen, die durch die Regulation der Expression der eingeführten Gene verstärkt wird.

13. Verfahren nach einem der Ansprüche 1 bis 12, wobei die blutbildenden Vorläuferzellen, die von den pluripotenten Stammzellen stammen, blutbildende Vorläuferzellen sind, die ein oder mehrere eingeführte Gene aufweisen, ausgewählt aus Genen der MYC-Familie, Bmi1-Genen, Genen der BCL2-Familie und Genen, die die p53-Genexpression unterdrücken, und eine proliferative und/oder differenzierende Fähigkeit aufweisen, die durch die Regulation der Expression der eingeführten Gene verstärkt wird.

14. Verfahren nach einem der Ansprüche 1 bis 13, wobei die sackartigen Strukturen durch eine Co-Kultur mit Feeder-Zellen bei einer Inkubationszeit von 14 bis 17 Tagen erhältlich sind.

## Revendications

1. Procédé pour produire des mégacaryocytes et/ou des plaquettes, comprenant
(i) la formation de cellules progénitrices hématopoïétiques *ex vivo* dans une structure analogue à un sac par différenciation de cellules souches pluripotentes induites (iPS) humaines, laquelle structure analogue à un sac est une structure sacciforme tridimensionnelle contenant des cellules progénitrices hématopoïétiques à l'intérieur ; et
(ii) la culture des cellules progénitrices hématopoïétiques séparées des cellules septales de la structure analogue à un sac *ex vivo* en présence d'un composé représenté par la formule (I), d'un tautomère, promédicament ou sel pharmaceutiquement acceptable du composé ou d'un solvate de celui-ci, pour que les cellules progénitrices hématopoïétiques deviennent différenciées en mégacaryocytes et/ou plaquettes : 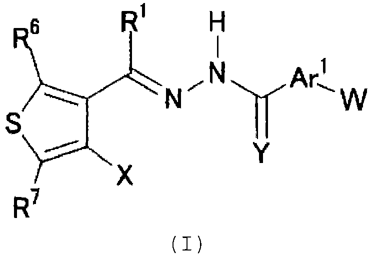
dans laquelle W est un substituant représenté par la formule (la) : 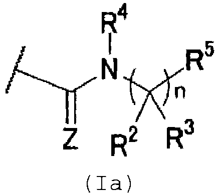
R¹ est un groupe méthyle,
chacun de R², R³, R⁴ et R⁶ est un atome d'hydrogène,
n est l'entier 1 ou 2,
R⁵ est un groupe phényle, un groupe 2-pyridyle, un groupe 3-pyridyle, un groupe 4-pyridyle, un groupe 3-pyridazinyle, un groupe 4-pyridazinyle, un groupe 2-pyrimidinyle, un groupe 4-pyrimidinyle, un groupe 5-pyrimidinyle, ou un groupe 2-pyrazinyle qui peut être substitué par un ou plusieurs substituants indépendamment représentés par V¹, sous réserve que, lorsque n vaut 2, R⁵ ne soit pas un groupe pyridyle non substitué,
R⁷ est un groupe phényle qui peut être substitué par un ou plusieurs substituants choisis parmi les groupes méthyle, les groupes t-butyle, les atomes d'halogène, les groupes méthoxy, les groupes trifluorométhyle et les groupes trifluorométhoxy, ou est un substituant représenté par l'une quelconque des formules (A01) à (A15) : 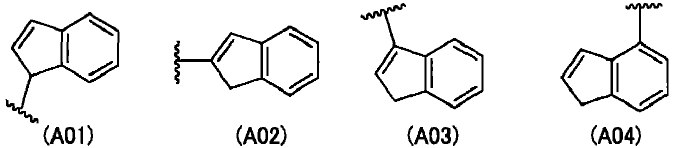 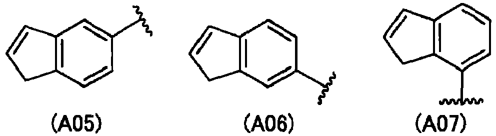 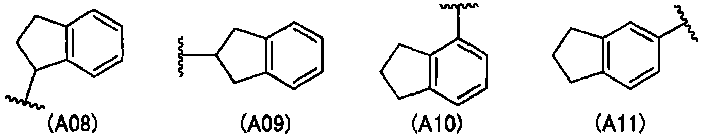 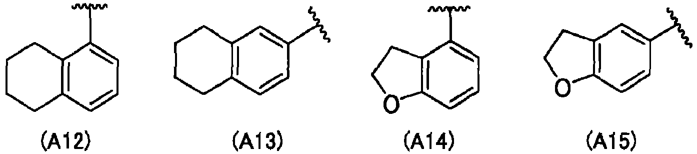
Ar¹ est représenté par la formule (IV) : 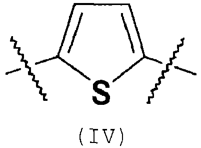
X est -OH,
chacun de Y et Z est indépendamment un atome d'oxygène ou un atome de soufre, et
V¹ est représenté par l'une quelconque des formules (V) à (XXII) :

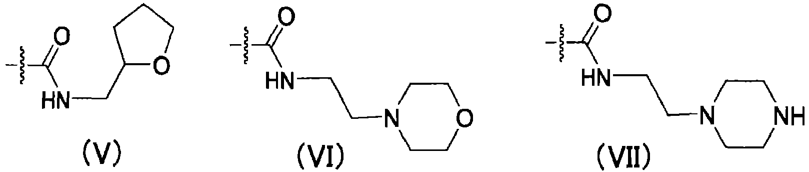

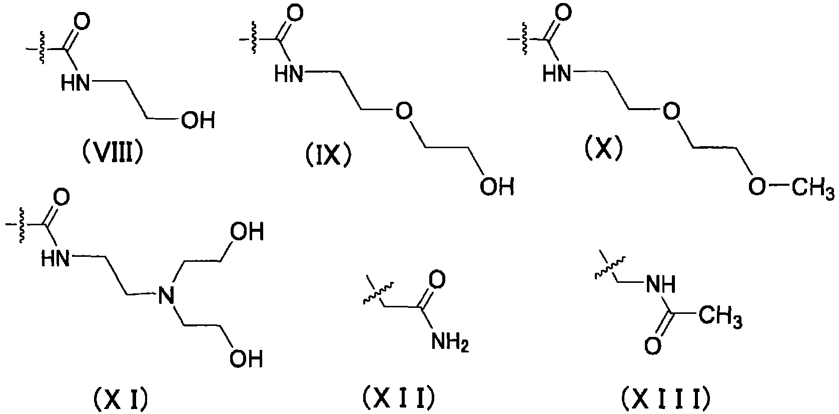

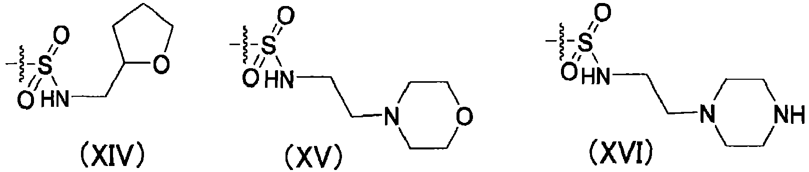

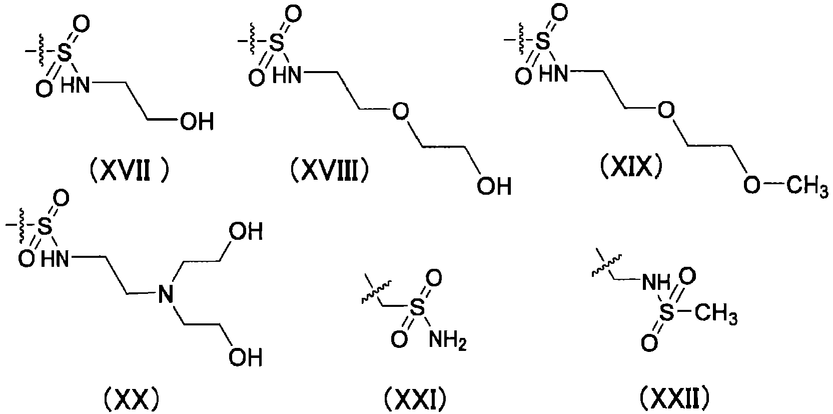

2. Procédé selon la revendication 1, dans lequel Y et Z sont des atomes d'oxygène.

3. Procédé selon la revendication 1, dans lequel R⁵ est un groupe phényle qui peut être substitué par un ou plusieurs substituants indépendamment représentés par V¹.

4. Procédé selon la revendication 1, dans lequel R⁵ est un groupe 4-pyridyle.

5. Procédé selon la revendication 1, dans lequel R⁵ est un groupe phényle substitué par un substituant représenté par la formule (VIII) :

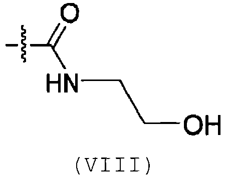

6. Procédé selon l'une quelconque des revendications 1 à 5, dans lequel n est l'entier 1.

7. Procédé selon l'une quelconque des revendications 1 à 6, dans lequel R⁷ est un groupe phényle substitué par un ou plusieurs substituants choisis parmi les groupes méthyle, les groupes t-butyle, les atomes d'halogène, les groupes méthoxy, les groupes trifluorométhyle et les groupes trifluorométhoxy.

8. Procédé selon l'une quelconque des revendications 1 à 6, dans lequel R⁷ est un groupe phényle qui peut être substitué par un ou deux atomes d'halogène.

9. Procédé selon la revendication 1, dans lequel le composé représenté par la formule (I) est le (E)-5-(2-{1-[5-(3,4-dichlorophényl)-4-hydroxythiophén-3-yl]-éthylidène}hydrazinecarbonyl)-N-[4-(2-hydroxyéthyl-carbamoyl)benzyl]thiophène-2-carboxamide, le (E)-5-(2-{1-[5-(4-bromophényl)-4-hydroxythiophén-3-yl]éthylidène}-hydrazinecarbonyl)-N-[4-(2-hydroxyéthylcarbamoyl)benzyl]-thiophène-2-carboxamide ou le (E)-5-(2-{1-[5-(3,4-dichlorophényl)-4-hydroxythiophén-3-yl]éthylidène}-hydrazinecarbonyl)-N-(pyridin-4-ylméthyl)thiophène-2-carboxamide.

10. Procédé selon la revendication 1, dans lequel n est l'entier 1, R⁵ est un groupe pyridyle, un groupe pyrazinyle ou un groupe phényle substitué par un substituant représenté par la formule (VII), (VIII), (XI) ou (XII) : 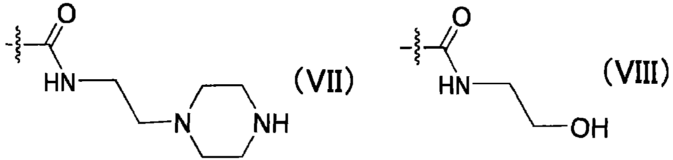 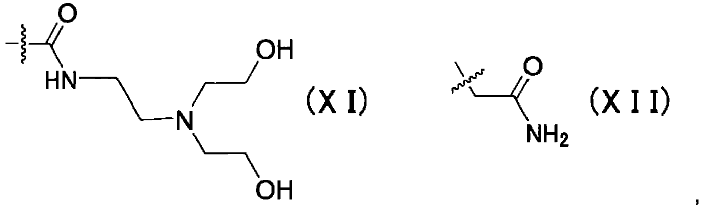 R⁷ est un groupe phényle qui peut être substitué par un ou deux atomes d'halogène ou un substituant représenté par la formule (A11), (A13) ou (A15) : 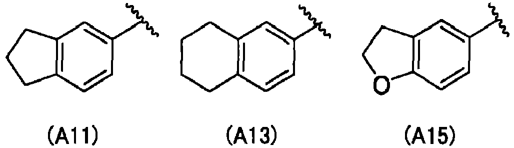 et Y et Z sont des atomes d'oxygène.

11. Procédé selon la revendication 1, dans lequel le composé représenté par la formule (I) est le (E)-5-(2-{1-[5-(3,4-dichlorophényl)-4-hydroxythiophén-3-yl]-éthylidène}hydrazinecarbonyl)-N-[4-(2-hydroxyéthyl-carbamoyl)benzyl]thiophène-2-carboxamide, le (E)-5-(2-{1-[5-(4-bromophényl)-4-hydroxythiophén-3-yl]éthylidène}-hydrazinecarbonyl)-N-[4-(2-hydroxyéthylcarbamoyl)benzyl]-thiophène-2-carboxamide, le (E)-5-(2-{1-[5-(3,4-dichloro-phényl)-4-hydroxythiophén-3-yl]éthylidène}hydrazine-carbonyl)-N-(pyridin-4-ylméthyl)thiophène-2-carboxamide, le (E)-2-(3,4-dichlorophényl)-4-[1-(2-{5-[(pyrazin-2-yl-méthyl)carbamoyl]thiophène-2-carbonyl}hydrazono)éthyl]-thiophén-3-olate de potassium, le (E)-5-(2-{1-[5-(4-bromo-phényl)-4-hydroxythiophén-3-yl]éthylidène}hydrazine-carbonyl)-N-{4-[2-(pipérazin-1-yl)éthylcarbamoyl]benzyl}-thiophène-2-carboxamide, le (E)-N-[4-(2-amino-2-oxoéthyl)-benzyl]-5-(2-{1-[5-(3,4-dichlorophényl)-4-hydroxythiophén-3-yl]éthylidène}hydrazinecarbonyl)thiophène-2-carboxamide ou le (E)-N-(4-{2-[bis(2-hydroxyéthyl)amino]éthyl-carbamoyl}benzyl)-5-(2-{1-[5-(4-t-butylphényl)-4-hydroxy-thiophén-3-yl]éthylidène}hydrazinecarbonyl)thiophène-2-carboxamide.

12. Procédé selon l'une quelconque des revendications 1 à 11, dans lequel les cellules progénitrices hématopoïétiques dérivées de cellules souches pluripotentes possèdent un ou plusieurs gènes introduits choisis parmi les oncogènes, les gènes Polycomb, les gènes suppresseurs d'apoptose et les gènes qui suppriment un gène suppresseur de tumeur, et présentent une capacité de prolifération et/ou de différenciation amplifiée par la régulation de l'expression des gènes introduits.

13. Procédé selon l'une quelconque des revendications 1 à 12, dans lequel les cellules progénitrices hématopoïétiques dérivées de cellules souches pluripotentes sont des cellules progénitrices hématopoïétiques qui possèdent un ou plusieurs gènes introduits choisis parmi les gènes de la famille MYC, les gènes Bmi1, les gènes de la famille BCL2 et les gènes qui suppriment l'expression du gène p53, et présentent une capacité de prolifération et/ou de différenciation amplifiée par la régulation de l'expression des gènes introduits.

14. Procédé selon l'une quelconque des revendications 1 à 13, dans lequel les structures analogues à un sac peuvent être obtenues par co-culture avec des cellules nourricières pendant un temps d'incubation de 14 à 17 jours.
